# EUROPEAN PATENT APPLICATION

(11) **EP 4 414 457 A1**
(43) Date of publication of application: **14.08.2024**
(21) Application number: 22878392.4
(22) Date of filing: 28.09.2022
(51) Int. Cl.: C12Q 1/6806, C12Q 1/6813, C12Q 1/6827, C12Q 1/6851, C12Q 1/686, C12Q 1/6883, C12N 15/12

(54) **METHOD FOR ANALYZING STRUCTURAL POLYMORPHISM, PRIMER PAIR SET, AND METHOD FOR DESIGNING PRIMER PAIR SET**

(30) Priority: 04.10.2021 WO PCT/JP2021/036697
(71) Applicant: The University of Tokyo, Bunkyo-ku, Tokyo 113-8654 (JP)
(72) Inventor: YOKOYAMA, Kazuaki, Tokyo 113-8654 (JP)
(74) Representative: Hasegawa, Kan
(86) International application number: PCT/JP2022/036170
(87) International publication number: WO 2023/058522

(57) **Abstract**

The present invention provides an analysis method suitable for each SV by utilizing a structural characteristic of the SV. The analysis method is a method for analyzing the presence/absence of a specific structural variation in a sample DNA including a genomic DNA, a free DNA, or a complementary DNA derived from a subject, and including a detection step of detecting the presence/absence of the specific structural variation by performing PCR using the sample DNA as a template, and using, as a primer, a primer pair set consisting of a first primer pair and a second primer pair.

## Description

### TECHNICAL FIELD

The present invention relates to a method for analyzing a structural variation, a primer pair set used in detection of a structural variation, and a method for designing the primer pair set.

### BACKGROUND ART

A structural variation (hereinafter also referred to as an SV) is a mutation with a size of 50 base pairs (bp) or more out of differences in genome between living bodies, and is a generic term for polymorphisms such as deletion, insertion, tandem duplication, and inversion. It has been revealed through a large number of studies that an SV can be a genetic factor of various diseases including diseases caused by somatic cell mutations such as cancers, and diseases such as development disorder and intellectual disability.

As a method for detecting an abnormality such as an SV, a gene panel testing has been conventionally employed (for example, Japanese Translation of PCT International Application Publication No. 2017-503527 (corresponding to US Patent Application Publication No. 2016/333420) . In the gene panel testing, several to several hundred mutant genes are antecedently registered as targets, and primer pair sets capable of amplifying these registered mutant genes are prepared. Then, an arbitrary sequence is amplified by PCR, and the amplified sequence is analyzed with a sequencer to detect an abnormality such as an SV.

For example, for a cancer patient, a large number of mutant genes antecedently registered are simultaneously checked by the gene panel testing to find which gene of the patient has a mutation, and thus, the patient can receive a treatment in accordance with his/her constitution and disease conditions.

### SUMMARY OF INVENTION

### Technical Problem

In recent years, however, owing to the appearance of a next generation sequencer (NGS), the whole genome can be analyzed at an individual level. As a result, various SVs have been consequently discovered, and the number is exponentially increasing. The structure of an SV varies among individuals, and it has been revealed, for example, that even when patients have the same disease derived from a mutation of the same gene, an SV in the gene differs therebetween.

In the gene panel testing, it is necessary to antecedently register mutant genes used as targets, and further to prepare primer pair sets capable of amplifying these genes, and hence, it is impossible to cope with all of a variety of SVs described above. Therefore, not a method for exhaustively testing a large number of patients but an SV test method optimized for every patient based on genetic information of the patient is demanded.

The present invention has been devised in consideration of the above-described problems, and an object is to provide an analysis method suitable for each SV by utilizing a structural characteristic of the SV.

### Means for Solving Problem

The present inventor has made earnest studies to solve the above-described problem, and as a result, has found that the problem can be solved, by utilizing a structural characteristic of an SV, by a method including a step of performing PCR using a primer pair set including a combination of a first primer pair for amplifying a wild-type nucleotide sequence not including a specific SV, and a second primer pair for amplifying a mutant nucleotide sequence including the specific SV, and thus, the present invention has been accomplished.

Specifically, the present invention is as follows.

[1] A method for analyzing the presence/absence of a specific structural variation in a sample DNA including a genomic DNA, a free DNA or a complementary DNA derived from a subject, comprising:
   a detection step of detecting the presence/absence of the specific structural variationbyperforming PCR using, as a template, the sample DNA, and using, as a primer, a primer pair set consisting of a first primer pair and a second primer pair,
   wherein the specific structural variation includes at least one selected from the group consisting of insertion, translocation, deletion, inversion, and tandem duplication, and the primer pair set is selected in accordance with the specific structural variation,
   the first primer pair consists of a forward primer (WT-F) and a reverse primer (WT-R) that anneal at positions for amplifying a target sequence in a wild-type nucleotide sequence not including the specific structural variation,
   the second primer pair consists of a forward primer (Mut-F) and a reverse primer (Mut-R) that anneal at positions for amplifying a target sequence in a mutant nucleotide sequence including the specific structural variation, and
   the primer pair set includes one or more selected from the group consisting of:
      a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
      a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
      a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
      a primer pair set (D) consisting of the first primer pair designed so that the length of the target sequence to be amplified by the forward primer (WT-F) and the reverse primer (WT-R) when the specific structural variation is included is twice or more than that when the specific structural variation is not included, and the second primer pair designed so that the target sequence to be amplified by the forward primer (Mut-F) and the reverse primer (Mut-R) is present only when the specific structural variation is included.
[2] The method according to [1], wherein when the specific structural variation is translocation occurring between two chromosomes, the forward primer (WT-F) and the reverse primer (WT-R) of the first primer pair in the primer pair set (B) anneal to only one of the two chromosomes.
[3] The method according to [1], wherein the forward primer (WT-F) of the first primer pair and the forward primer (Mut-F) of the second primer pair are common primers having the same nucleotide sequence, or the reverse primer (WT-R) of the first primer pair and the reverse primer (Mut-R) of the second primer pair are common primers having the same nucleotide sequence.
[4] The method according to [1], wherein the PCR performed in the detection step is quantitative PCR in which an amplification product is quantitatively determined using a nucleic acid probe that binds to a target sequence and has a labeling function, and the quantitative PCR is real-time PCR or digital PCR.
[5] The method according to [4], wherein the nucleic acid probe includes one or more nucleic acid probe sets each consisting of a first probe that hybridizes to the target sequence of the first primer pair, and a second probe that hybridizes to the target sequence of the second primer pair, the first probe and the second probe of the nucleic acid probe set are labeled by different fluorescent labels, and in the quantitative PCR, an amount of amplification of the target sequence of the first primer pair and an amount of amplification of the target sequence of the second primer pair are both quantitatively determined by detecting fluorescence derived from the first probe and fluorescence derived from the second probe.
[6] The method according to [5], wherein at least a part of the first probe is designed to hybridize to a region, in the target sequence of the first primer pair, having a sequence different from the target sequence of the second primer pair, and at least a part of the second probe is designed to hybridize to a region, in the target sequence of the second primer pair, having a sequence different from the target sequence of the first primer pair.
[7] The method according to [6], comprising a calculation step of calculating a ratio between the wild-type nucleotide sequence and the mutant nucleotide sequence by comparing the amount of amplification of the target sequence of the first primer pair and the amount of amplification of the target sequence of the second primer pair based on results of the quantitative determination by the PCR.
[8] The method according to [5], wherein when two or more primer pair sets and two or more nucleic acid probe sets are used in the quantitative PCR, the primer pair sets and the nucleic acid probe sets are in a relationship of one-to-one correspondence.
[9] The method according to [1], comprising, when the sample DNA includes the genomic DNA or the free DNA, before the detection step, a whole genome sequence analysis step of performing whole genome sequence analysis on the sample DNA, and a determination step of determining the specific structural variation based on a result of the whole genome sequence analysis step.
[10] The method according to [1], comprising, when the sample DNA includes the genomic DNA or the free DNA, a step of extracting the genomic DNA from the subject, or a step of isolating the free DNA from the subject.
[11] The method according to [1], comprising a monitoring step of obtaining subjects from one individual over time, and monitoring change over time of a state of the specific structural variation of the individual by analyzing the present/absence of the specific structural variation in each sample DNA obtained from the subject.
[12] The method according to [1], wherein the subject includes blood, bone marrow, a body cavity fluid, a living body tissue, or a lymph tissue of a human or an animal.
[13] The method according to [1], wherein the specific structural variation is a driver mutation of a disease.
[14] The method according to any one of [1] to [13], wherein sequencing of an amplification product obtained by the PCR is not performed.
[15] An MRD analysis method for analyzing minimal residual disease (MRD) by employing the method according to [1].
[16] A primer pair set for use in PCR for analyzing the presence/absence of a specific structural variation in a sample DNA including a genomic DNA, a free DNA, or a complementary DNA derived from a subject,
   the specific structural variation including at least one selected from the group consisting of insertion, translocation, deletion, inversion, and tandem duplication,
   the primer pair set comprising a first primer pair and a second primer pair,
   wherein the first primer pair consists of a forward primer (WT-F) and a reverse primer (WT-R) that anneal at positions for amplifying a target sequence in a wild-type nucleotide sequence not including the specific structural variation,
   the second primer pair consists of a forward primer (Mut-F) and a reverse primer (Mut-R) that anneal at positions for amplifying a target sequence in a mutant nucleotide sequence including the specific structural variation, and
   the primer pair set is one or more selected from the group consisting of:
      a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
      a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
      a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
      a primer pair set (D) consisting of the first primer pair designed so that the length of the target sequence to be amplified by the forward primer (WT-F) and the reverse primer (WT-R) when the specific structural variation is included is twice or more than that when the specific structural variation is not included, and the second primer pair designed so that the target sequence to be amplified by the forward primer (Mut-F) and the reverse primer (Mut-R) is present only when the specific structural variation is included.
[17] A method for designing a primer pair set for use in PCR for analyzing the presence/absence of a specific structural variation in a sample DNA including a genomic DNA, a free DNA, or a complementary DNA derived from a subject,
   wherein the specific structural variation includes at least one selected from the group consisting of insertion, translocation, deletion, inversion, and tandem duplication,
   the primer pair set comprises a first primer pair and a second primer pair,
   the first primer pair consists of a forward primer (WT-F) and a reverse primer (WT-R) that anneal at positions for amplifying a target sequence in a wild-type nucleotide sequence not including the specific structural variation,
   the second primer pair consists of a forward primer (Mut-F) and a reverse primer (Mut-R) that anneal at positions for amplifying a target sequence in a mutant nucleotide sequence including the specific structural variation, and
   the primer pair set is one or more selected from the group consisting of:
      a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
      a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
      a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
      a primer pair set (D) consisting of the first primer pair designed so that the length of the target sequence to be amplified by the forward primer (WT-F) and the reverse primer (WT-R) when the specific structural variation is included is twice or more that when the specific structural variation is not included, and the second primer pair designed so that the target sequence to be amplified by the forward primer (Mut-F) and the reverse primer (Mut-R) is present only when the specific structural variation is included.

### BRIEF DESCRIPTION OF DRAWINGS

Fig. 1 is a diagram illustrating an exemplified case in which a method of the present embodiment is applied when a structural variation is insertion.
Fig. 2 is a diagram illustrating a case 1 in which the method of the present embodiment is applied when the structural variation is translocation.
Fig. 3 is a diagram illustrating a case 2 in which the method of the present embodiment is applied when the structural variation is translocation.
Fig. 4 is a diagram illustrating a case 1 in which the method of the present embodiment is applied when the structural variation is inversion.
Fig. 5 is a diagram illustrating a case 2 in which the method of the present embodiment is applied when the structural variation is inversion.
Fig. 6 is a diagram illustrating a case 1 in which the method of the present embodiment is applied when the structural variation is deletion.
Fig. 7 is a diagram illustrating a case 2 in which the method of the present embodiment is applied when the structural variation is deletion.
Fig. 8 is a diagram illustrating an exemplified case in which the method of the present embodiment is applied when the structural variation is tandem duplication.
Fig. 9 is a diagram for explaining Example 1.
Fig. 10 is a diagram illustrating results of Example 1.
Fig. 11 is a diagram illustrating results obtained by performing a method of Example 1 a plurality of times over time.
Fig. 12 is a diagram for explaining Example 2.
Fig. 13 is a diagram illustrating results of Example 2.
Fig. 14 is a diagram for explaining Example 3.
Fig. 15 is a diagram illustrating results of Example 3.
Fig. 16 is a diagram for explaining Example 4.
Fig. 17 is a diagram illustrating results of Example 4.

### DESCRIPTION OF EMBODIMENTS

One embodiment of the present invention is a method for analyzing presence/absence of a specific structural variation in a sample DNA including a genomic DNA, a free DNA, or a complementary DNA derived from a subject, the method including a detection step of detecting the presence/absence of the specific structural variation by performing PCR using the sample DNA as a template, and using a primer pair set including a first primer pair and a second primer pair, wherein the specific structural variation includes at least one or more selected from the group consisting of insertion, translocation, deletion, inversion, and tandem duplication, the primer pair set is selected in accordance with the specific structural variation, the first primer pair consists of a forward primer (WT-F) and a reverse primer (WT-R) that anneal at positions for amplifying a target sequence in a wild-type nucleotide sequence not including the specific structural variation, and the second primer pair consists of a forward primer (Mut-F) and a reverse primer (Mut-R) that anneal at positions for amplifying a target sequence in a mutant nucleotide sequence including the specific structural variation.

Besides, the primer pair set includes one or more selected from the group consisting of:
a primer pair set (A) including the first primer pair designed so that the length of the target sequence to be amplified by the forwardprimer (WT-F) and the reverse primer (WT-R) when the specific structural variation is included may be 10 times or more than that when the specific structural variation is not included, and the second primer pair designed so that the target sequence to be amplified by the forward primer (Mut-F) and the reverse primer (Mut-R) may be present only when the specific structural variation is included;
a primer pair set (B) including the first primer pair designed so that the target sequence to be amplified by the forward primer (WT-F) and the reverse primer (WT-R) may be present only when the specific structural variation is not included, and the second primer pair designed so that the target sequence to be amplified by the forward primer (Mut-F) and the reverse primer (Mut-R) may be present only when the specific structural variation is included;
a primer pair set (C) including the first primer pair designed so that the target sequence to be amplified by the forward primer (WT-F) and the reverse primer (WT-R) may be present only when the specific structural variation is not included, and the second primer pair designed so that the length of the target sequence to be amplified by the forward primer (Mut-F) and the reverse primer (Mut-R) when the specific structural variation is included may be 1/10 or less than that when the specific structural variation is not included; and
a primer pair set (D) including the first primer pair designed so that the length of the target sequence to be amplified by the forwardprimer (WT-F) and the reverse primer (WT-R) when the specific structural variation is included may be twice or more than that when the specific structural variation is not included, and the second primer pair designed so that the target sequence to be amplified by the forward primer (Mut-F) and the reverse primer (Mut-R) may be present only when the specific structural variation is included.

### [Structural Variation]

A structural variation is a mutation with a size of 50 base pairs (bp) or more out of differences in genome between living bodies. The types of the structural variation include, in accordance with mutation patterns, insertion in which a different nucleotide sequence is inserted in a specific position, translocation in which a part of a nucleotide sequence is moved from a normal position to another position or another chromosome, deletion in which a part of a nucleotide sequence is deleted, inversion in which a part of a region is inversely converted, and tandem duplication in which a part of a region is duplicatively inserted. The specific structural variation of the present embodiment may be selected from the group consisting of insertion, translocation, deletion, inversion, and tandem duplication. Besides, a structural variation can be a driver mutation, that is, a gene mutation that can be a direct cause of occurrence or exacerbation of cancer, and hence, the specific structural variation may be a driver mutation of a disease of a patient also in the present embodiment.

### [Sample DNA]

The sample DNA in the present embodiment means a genomic DNA, a free DNA, or a complementary DNA derived from a subject. The subject herein is not especially limited as long as it is a subject obtained from a human or an animal, and from which a genomic DNA, a free DNA, or an RNA usable as a template of a complementary DNA can be collected. The subject is, however, preferably blood, bone marrow, a body cavity fluid, a living body tissue, or a lymph tissue of a human or an animal because such a subject can be easily collected. Examples of the animal include a dog, a pig, sheep, a goat, a bovine, and a mouse. A method for obtaining a genomic DNA, a free DNA, or a complementary DNA derived from the subject will be described later regarding an extraction step.

Herein, the term "genomic DNA" means a genetic substance found in the nuclear of the cell of the subject. The genomic DNA encompasses all genes, particularly a coding sequence and a regulatory element thereof. Herein, the term "free DNA" refers to a genetic substance that can be present in blood or a body cavity fluid, and has a part or the whole of genomic information, and examples include a cell-free DNA (cfDNA), and a circulating tumor DNA (ctDNA) . Herin, the term "blood" encompasses all of whole blood, a serum, and a plasma. Herein, the term "complementary DNA (cDNA) " means a DNA synthesized with a reverse transcriptase by using an RNA as a template.

### [PCR]

In PCR (polymerase chain reaction) in the present embodiment, any of known reagents can be selectively used as reagents necessary for PCR, such as a nucleic acid amplification enzyme (polymerase, particularly a DNA polymerase) and a dNTP mix. Besides, for example, the number of amplification cycles can be appropriately set in accordance with a nucleic acid amplification enzyme to be used, the length of an amplified nucleic acid, and the like. For example, the number of amplification cycles can be 10 to 100 cycles, and the upper limit of this range may be 90, 80, 70, 60, 50, or 40, and the lower limit of the range may be 15, 20, 25, 30, or 35.

The length of a region to be amplified by the PCR, namely, the length of the target sequence of the forward primer and the reverse primer of the primer pair, is not especially limited but can be appropriately set as long as the region to be amplified can be amplified. The length may be, for example, 50 to 1000 pb (base pairs), may be 100 to 800 bp, may be about 150 to 500 bp, or may be about 100 to 500 bp.

Besides, the nucleotide length of each primer is not especially limited as long as it is in the range capable of amplifying the region to be amplified, and may be a nucleotide length of, for example, 15 to 50, or about 20 to 40.

Now, a method of the present embodiment for analyzing presence/absence of a specific structural variation, and a method for designing a primer pair set used in the method will be described.

The method of the present embodiment for analyzing presence/absence of a specific structural variation includes a detection step of detecting the presence/absence of the specific structural variation based on a result of PCR performed using a sample DNA as a template, and using a primer pair set including two types of primer pairs of a first primer pair and a second primer pair. Herein, a method for simultaneously amplifying a plurality of gene regions by simultaneously using two or more primer pairs in one PCR reaction system is also designated as multiplex PCR.

In the present embodiment, a primer pair refers to a pair of primers used in PCR for amplifying a target nucleotide sequence, and consisting of one forward primer and one reverse primer. In PCR, the forward primer and the reverse primer anneal complementarily to one DNA strand (antisense strand) and the other DNA strand (sense strand), respectively of a double-stranded DNA used as a template.

Besides, herein, a nucleotide sequence to be amplified by the PCR using the forward primer and the reverse primer paired thereto is designated as a target sequence. In other words, a nucleotide sequence to be amplified by the PCR using the primer pair is designated as a target sequence. Besides, the forward primer and the reverse primer of the first primer pair are designated also as WT-F and WT-R, respectively, and the forward primer and the reverse primer of the second primer pair are designated also as Mut-F and Mut-R, respectively.

### [PCR Amplification of Sample DNA]

As described above, in the PCR of the present embodiment, the target sequence is amplified by using a sample DNA as a template, and using the primer pair set consisting of two types of primer pairs of the first primer pair and the second primer pair. It is noted that one primer pair set may be used, or two or more primer pair sets may be used in the PCR.

### (First Primer Pair and Second Primer Pair)

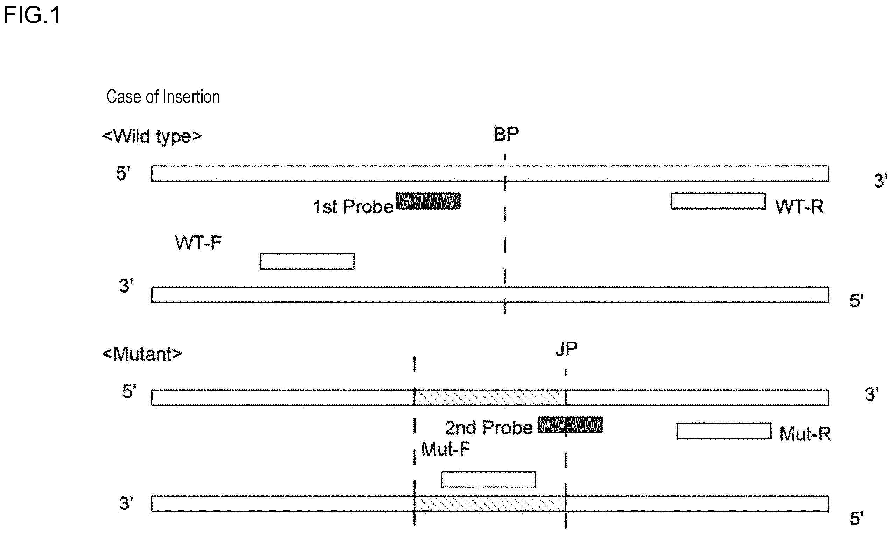

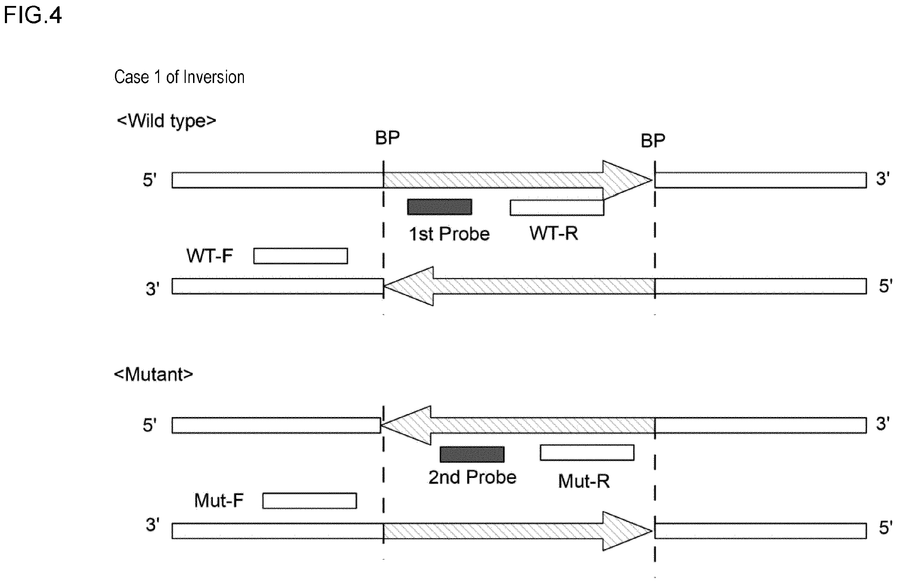

The first primer pair is used for detecting a nucleotide sequence not including the specific structural variation (wild-type nucleotide sequence (hereinafter also referred to as "Wild Type")) in the sample DNA, and is designed to allow the forward primer (WT-F) and the reverse primer (WT-R) to sandwich a nucleotide sequence including a position or a part or the whole of a region corresponding to the specific structural variation in the wild-type nucleotide sequence. In other words, the target sequence of the forward primer (WT-F) and the reverse primer (WT-R) includes a portion of the wild-type nucleotide sequence corresponding to the specific structural variation, or a part or the whole of a region corresponding to the specific structural variation. Here, a portion corresponding to the specific structural variation or a region corresponding to the specific structural variation means a location in the wild-type nucleotide sequence corresponding to a portion or a region where the structural variation occurs in a sample DNA including the specific structural variation (mutant nucleotide sequence) . More specifically, it refers to, for example, BPs (breakpoints of a nucleotide sequence) in "Wild type" illustrated in Figs. 1 to 3, or a region sandwiched between between BPs or TPs of "Wild type" illustrated in Figs. 4 to 8 described later.

The second primer pair is used for detecting a nucleotide sequence including the specific structural variation (mutant nucleotide sequence (hereinafter also referred to as "Mutant")) in the sample DNA, and is designed so as to have the forward primer (Mut-F) and the reverse primer (Mut-R) to sandwich a nucleotide sequence including a position or a part or the whole of a region in the mutant nucleotide sequence where the specific structural variation occurs. In other words, the target sequence of the forward primer (Mut-F) and the reverse primer (Mut-R) includes a portion in the mutant nucleotide sequence where the specific structural variation occurs, or a part or the whole of a region where the specific structural variation occurs.

### (Common Primer)

One primer of the first primer pair and one primer of the second primer pair may be common primers having the same nucleotide sequence. More specifically, the forwardprimer (WT-F) of the first primer pair and the forward primer (Mut-F) of the second primer pair may be common primers having the same nucleotide sequence, or the reverse primer (WT-R) of the first primer pair and the reverse primer (Mut-R) of the second primer pair may be common primers having the same nucleotide sequence. When common primers are used, the number of primers to be created can be reduced, which can lead to simplification of an experimental method and cost reduction. Besides, the common primers may be designed to bind to a nucleotide sequence not including a region where the specific structural variation is present in the sample DNA.

### (Primer Pair Set)

The detection step of detecting presence/absence of a specific structural variation of the present embodiment uses, in performing PCR, the primer pair set including the first primer pair and the second primer pair. At this point, one or more primer pair sets are selected in accordance with the type of the specific structural variation. Here, the type of the specific structural variation is any one selected from the group consisting of insertion, translocation, deletion, inversion, and tandem duplication.

Now, the primer pair sets (A) to (D) in accordance with the type of the specific structural variation are specifically described with reference to Figs. 1 to 8. It is noted that combination patterns of the first primer pair and the second primer pair included in the following primer pair sets are shown only as examples, and the combination is not limited thereto.

Here, "Wild type" of each drawing means a wild-type nucleotide sequence not including the specific structural variation in the sample DNA, and "Mutant" means a mutant nucleotide sequence including the specific structural variation in the sample DNA. Besides, the forward primer of the first primer pair is shown as "WT-F" and the reverse primer thereof is shown as "WT-R", and the forward primer of the second primer pair is shown as "Mut-F" and the reverse primer thereof is shown as "Mut-R". Besides, "BP" illustrated in each drawing corresponds to a breakpoint of a nucleotide sequence, and when a nucleotide sequence is broken at a breakpoint of the "Wild type", the structural variation can be caused.

### (Primer Pair Set (A))

The primer pair set (A) includes the first primer pair designed so that the length of a nucleotide sequence sandwiched between the forward primer (WT-F) and the reverse primer (WT-R) when the specific structural variation is included may be 10 times or more than that when the specific structural variation is not included, and the second primer pair designed so that a nucleotide sequence sandwiched between the forward primer (Mut-F) and the reverse primer (Mut-R) may be present only when the specific structural variation is included. In other words, the primer pair set (A) includes the first primer pair designed so that the length of the target sequence to be amplified by the forward primer (WT-F) and the reverse primer (WT-R) when the specific structural variation is included may be 10 times or more than that when the specific structural variation is not included, and the second primer pair designed so that the target sequence to be amplified by the forward primer (Mut-F) and the reverse primer (Mut-R) may be present only when the specific structural variation is included.

The primer pair set (A) can be selected when the specific structural variation is insertion. The primer pair set (A) will now be described by exemplifying insertion illustrated in Fig. 1. In Fig. 1, a region where insertion occurs in "Mutant" (hereinafter referred to as the insertion region) is cross-hatched.

In the first primer pair, the forward primer (WT-F) and the reverse primer (WT-R) are designed so as to sandwich a portion corresponding to the specific structural variation in "Wild type", namely, the breakpoint ("BP" in the drawing) where the nucleotide sequence of "Wild type" is broken on the occurrence of insertion. Besides, the first primer pair is designed so that the length of a nucleotide sequence sandwiched between the forward primer (WT-F) and the reverse primer (WT-R) when the first primer pair anneals to "Mutant" may be 10 times or more than that when the first primer pair anneals (also designated as "hybridize") to "Wild type". Owing to this design, when the template is "Mutant", the target sequence is not amplified by the first primer pair in the PCR, or amplification efficiency of the target sequence is reduced as compared with that when the template is "Wild type".

The second primer pair can be designed so as to allow one primer (P1) of the forward primer (Mut-F) and the reverse primer (Mut-R) to anneal to a nucleotide sequence including the insertion region of "Mutant". In this case, the primer may be designed to anneal to the insertion region, or may be designed to anneal to a portion across the insertion region and another nucleotide sequence. In this case, the other primer (P2) of the second primer pair may be designed to anneal to any position in "Mutant" as long as the target sequence of the primer (P1) and the primer (P2) can be amplified by the PCR.

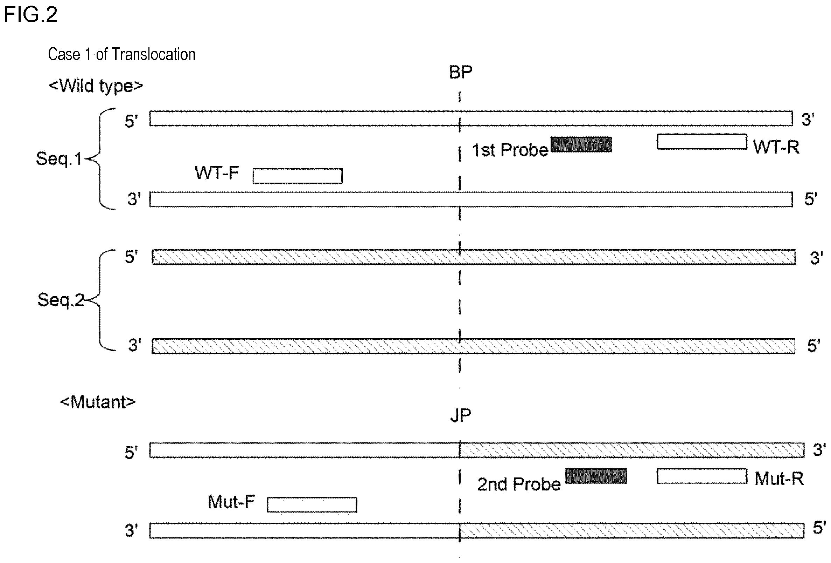

More specifically, the forward primer (Mut-F) may be designed to anneal to the insertion region of "Mutant", and the reverse primer (Mut-R) may be designed to anneal to a region not including the insertion region of "Mutant" as shown Fig. 2. When these are thus designed, a nucleotide sequence sandwiched between the forward primer (Mut-F) and the reverse primer (Mut-R) of the second primer pair is present only when the specific structural variation is included, and therefore, the target sequence can be amplified only when the template is "Mutant".

When the primer pair set (A) including the first primer pair and the second primer pair described above is used, for example, both a case where insertion of the structural variation occurs and a case where it does not occur can be detected by performing PCR once.

In the case illustrated in Fig. 1, the reverse primer (WT-R) of the first primer pair and the reverse primer (Mut-R) of the second primer pair designed to anneal to a nucleotide sequence not including the insertion region may be common primers having the same nucleotide sequence.

### (Primer Pair Set (B))

The primer pair set (B) includes a first primer pair designed so that a nucleotide sequence sandwiched between the forward primer (WT-F) and the reverse primer (WT-R) may be present only when the specific structural variation is not included, and a second primer pair designed so that a region sandwiched between the forward primer (Mut-F) and the reverse primer (Mut-R) may be present only when the specific structural variation is included. In other words, the primer pair set (B) includes the first primer pair designed so that the target sequence to be amplified by the forward primer (WT-F) and the reverse primer (WT-R) may be present only when the specific structural variation is not included, and the second primer pair designed so that the target sequence to be amplified by the forward primer (Mut-F) and the reverse primer (Mut-R) may be present only when the specific structural variation is included. The primer pair set (B) can be selected when the specific structural variation is translocation or inversion.

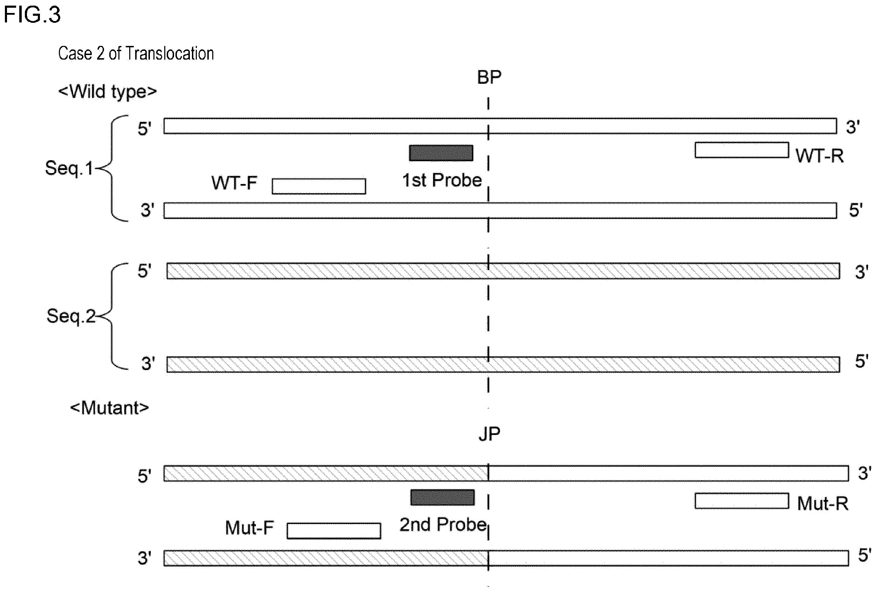

The primer pair set (B) will be first described by exemplifying translocation illustrated in Figs. 2 and 3. In Figs. 2 and 3, one of two sequences ("Seq. 1" and "Seq. 2" in the drawings) involved in translocation is cross-hatched for description convenience.

As illustrated in Figs. 2 and 3, the first primer pair can be designed so that a portion corresponding to the specific structural variation in "Wild type", namely, a breakpoint (BP, illustrated with a broken line in the drawings) where the nucleotide sequence of "Wild type" is broken when translocation occurs, may be sandwiched between the forward primer (WT-F) and the reverse primer (WT-R). Owing to this design, a nucleotide sequence sandwiched between the forward primer (WT-F) and the reverse primer (WT-R) is present only when the template is "Wild type", and the first primer pair can amplify the target sequence only in "Wild type".

On the other hand, the second primer pair can be designed, in the case illustrated in Fig. 2, so as to allow the forward primer (Mut-F) to anneal to a sequence corresponding to "Seq. 1" in "Mutant", and the reverse primer (Mut-R) to anneal to a sequence corresponding to the other "Seq. 2". In the case of Fig. 3, the second primer pair can be designed so as to allow the forward primer (Mut-F) to anneal to a sequence corresponding to "Seq. 2" in "Mutant", and the reverse primer (Mut-R) to anneal to a sequence corresponding to "Seq. 1". Owing to this design, only when the specific structural variation is included, a region sandwiched between the forward primer (Mut-F) and the reverse primer (Mut-R) of the second primer pair is present , and the target sequence can be amplified only when the template is "Mutant".

When the primer pair set (B) including the first primer pair and the second primer pair described above is used, both a case where translocation of the structural variation occurs and a case where it does not occur can be detected at one time.

Besides, when the specific structural variation is translocation occurring between two chromosomes as described above, the forward primer (WT-F) and the reverse primer (WT-R) of the first primer pair can be designed so as to anneal to only one of the two chromosomes. When the first primer pair is thus designed, a case where translocation of the structural variation occurs and a case where it does not occur can be more definitely distinguishably detected by the PCR.

Here, in the exemplified case of Fig. 2, the forward primer (WT-F) of the first primer pair and the forward primer (Mut-F) of the second primer pair may be common primers having the same nucleotide sequence, and in the exemplified case of Fig. 3, the reverse primer (WT-R) of the first primer pair and the reverse primer (Mut-R) of the second primer pair may be common primers having the same nucleotide sequence.

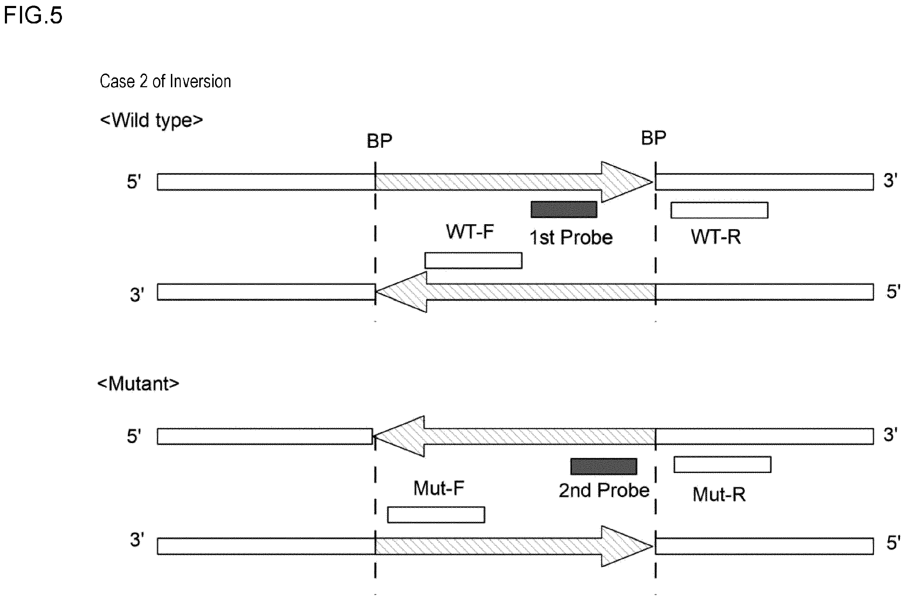

Next, the primer pair set (B) will be described by exemplifying inversion illustrated in Figs. 4 and 5. In Figs. 4 and 5, for description convenience, regions where inversion occurs are cross-hatched, and the direction of each sequence is illustrated with an arrow.

As illustrated in Figs. 4 and 5, the first primer pair is designed to allow one primer of the forward primer (WT-F) and the reverse primer (WT-R) to anneal to a region in "Wild type" corresponding to a region where inversion occurs in "Mutant" (region sandwiched between BPs in the drawings), and the other primer to anneal to a region where inversion does not occur. Owing to this design, a nucleotide sequence sandwiched between the forward primer (WT-F) and the reverse primer (WT-R) is present only when the specific structural variation is not included, and the target sequence can be amplified only when the template is "Wild type". Fig. 4 illustrates an exemplified case where the forward primer (WT-F) is designed to anneal to a region where inversion does not occur, and the reverse primer (WT-R) is designed to anneal to a region corresponding to occurrence of inversion. Fig. 5 illustrates an exemplified case where the forward primer (WT-F) is designed to anneal to a region corresponding to occurrence of inversion and the reverse primer (WT-R) is designed to anneal to a region where inversion does not occur.

On the other hand, the second primer pair is designed to allow one primer of the forward primer (Mut-F) and the reverse primer (Mut-R) to anneal to a nucleotide sequence where inversion occurs, and the other primer to anneal to a region where inversion does not occur. Owing to this design, only when the specific structural variation is included, a nucleotide sequence sandwiched between the forward primer (Mut-F) and the reverse primer (Mut-R) is present, and the target sequence can be amplified only when the template is "Mutant". Fig. 4 illustrates an exemplified case where the forward primer (Mut-F) is designed to anneal to a region where inversion does not occur, and the reverse primer (Mut-R) is designed to anneal to a region where inversion occurs. Fig. 5 illustrates an exemplified case where the forward primer (Mut-F) is designed to anneal to a region where inversion occurs, and the reverse primer (Mut-R) is designed to anneal to a region where inversion does not occur.

When the primer pair set (B) including the first primer pair and the second primer pair described above is used, both a case where translocation or inversion of the structural variation occurs and a case where it does not occur can be detected by the PCR at one time.

Here, in the exemplified case of Fig. 4, the forward primer (WT-F) of the first primer pair and the forward primer (Mut-F) of the second primer pair may be common primers having the same nucleotide sequence, and in the exemplified case of Fig. 5, the reverse primer (WT-R) of the first primer pair and the reverse primer (Mut-R) of the second primer pair may be common primers having the same nucleotide sequence.

### (Primer Pair Set (C))

The primer pair set (C) includes the first primer pair designed so that a region sandwiched between the forward primer (WT-F) and the reverse primer (WT-R) may be present only when the specific structural variation is not included, and the second primer pair designed so that the length of a nucleotide sequence sandwiched between the forward primer (Mut-F) and the reverse primer (Mut-R) when the specific structural variation is included as compared may be 1/10 or less than that when the specific structural variation is not included. In other words, the primer pair set (C) includes the first primer pair designed so that the target sequence to be amplified by the forward primer (WT-F) and the reverse primer (WT-R) may be present only when the specific structural variation is not included, and the second primer pair designed so that the length of the target sequence to be amplified by the forward primer (Mut-F) and the reverse primer (Mut-R) when the specific structural variation is included may be 1/10 or less than that when the specific structural variation is not included. The primer pair set (C) can be selected when the specific structural variation is deletion.

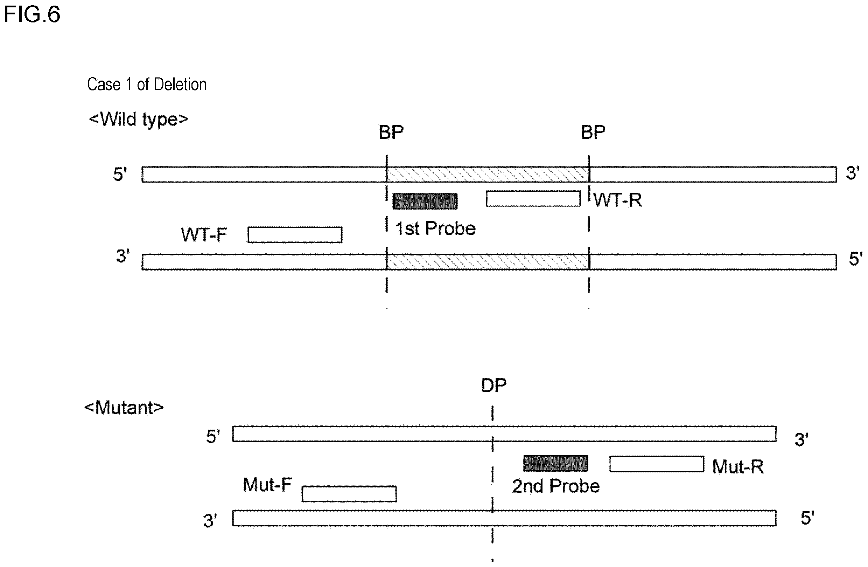

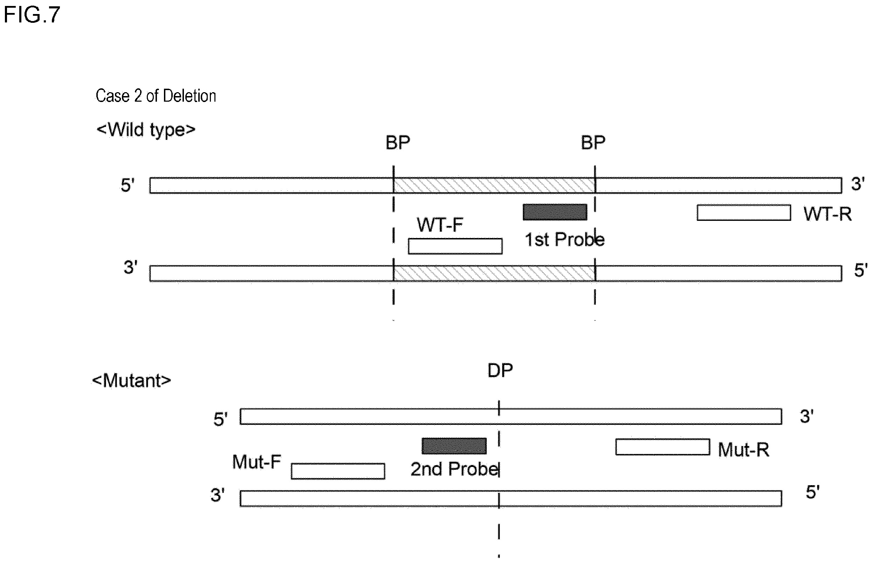

The primer pair set (C) will now be described by exemplifying deletion illustrated in Figs. 6 and 7. In Figs. 6 and 7, a region cross-hatched in "Wild type" is deleted in "Mutant".

As illustrated in Figs. 6 and 7, the first primer pair is designed to allow one primer of the forward primer (WT-F) and the reverse primer (WT-R) to anneal to a region in "Wild type" corresponding to a region where deletion occurs in "Mutant" (region sandwiched between BPs in the drawings), and the other primer to anneal to a region where deletion does not occur. Owing to this design, a nucleotide sequence sandwiched between the forward primer (WT-F) and the reverse primer (WT-R) is present only when the specific structural variation is not included, and the target sequence of the first primer pair can be amplified only when the template is "Wild type". Fig. 6 illustrates an exemplified case where the forward primer (WT-F) is designed to anneal to a region where deletion does not occur and the reverse primer (WT-R) is designed to anneal to a region corresponding to occurrence of deletion. Fig. 7 illustrates an exemplified case where the forward primer (WT-F) is designed to anneal to a region corresponding to occurrence of deletion and the reverse primer (WT-R) is designed to anneal to a region where deletion does not occur. It is noted that the first primer pair may be designed to allow both the forward primer (WT-F) and the reverse primer (WT-R) to anneal to a region in "Wild type" corresponding to a region where deletion occurs in "Mutant" (region sandwiched between BPs in the drawing).

On the other hand, in the second primer pair, the forward primer (Mut-F) and the reverse primer (Mut-R) are designed so as to sandwich a portion where deletion occurs (DP in the drawing) . The second primer pair can anneal to a nucleotide sequence of a case where deletion does not occur, namely, a case where the specific structural variation is not included, but the second primer pair is designed so that the length of the nucleotide sequence sandwiched between the forward primer (Mut-F) and the reverse primer (Mut-R) when deletion occurs may be 1/10 or less than that when deletion does not occur. Thus, the region sandwiched between the forward primer (Mut-F) and the reverse primer (Mut-R) of the second primer pair is longer when deletion does not occur, and therefore, the target sequence is not amplified by the PCR, or the amplification efficiency of the target sequence is reduced. On the other hand, when deletion occurs, the target sequence of the forward primer (Mut-F) and the reverse primer (Mut-R) can be efficiently amplified by the PCR.

When the primer pair set (C) including the first primer pair and the second primer pair described above is used, both a case where deletion of the structural variation occurs and a case where it does not occur can be detected by the PCR at one time.

Here, in the exemplified case of Fig. 6, the forward primer (WT-F) of the first primer pair and the forward primer (Mut-F) of the second primer pair may be common primers having the same nucleotide sequence, and in the exemplified case of Fig. 7, the reverse primer (WT-R) of the first primer pair and the reverse primer (Mut-R) of the second primer pair may be common primers having the same nucleotide sequence.

### (Primer Pair Set (D))

The primer pair set (D) includes a first primer pair designed so that the length of a nucleotide sequence sandwiched between the forward primer (WT-F) and the reverse primer (WT-R) when the specific structural variation is included may be twice or more than that when the specific structural variation is not included, and a second primer pair designed so that a nucleotide sequence sandwiched between the forward primer (Mut-F) and the reverse primer (Mut-R) may be present only when the specific structural variation is included. In other words, the primer pair set (D) includes the first primer pair designed so that the length of the target sequence to be amplified by the forward primer (WT-F) and the reverse primer (WT-R) when the specific structural variation is included may be twice or more than as that when the specific structural variation is not included, and the second primer pair designed so that the target sequence to be amplified by the forward primer (Mut-F) and the reverse primer (Mut-R) may be present only when the specific structural variation is included. The primer pair set (D) can be selected when the specific structural variation is tandem duplication.

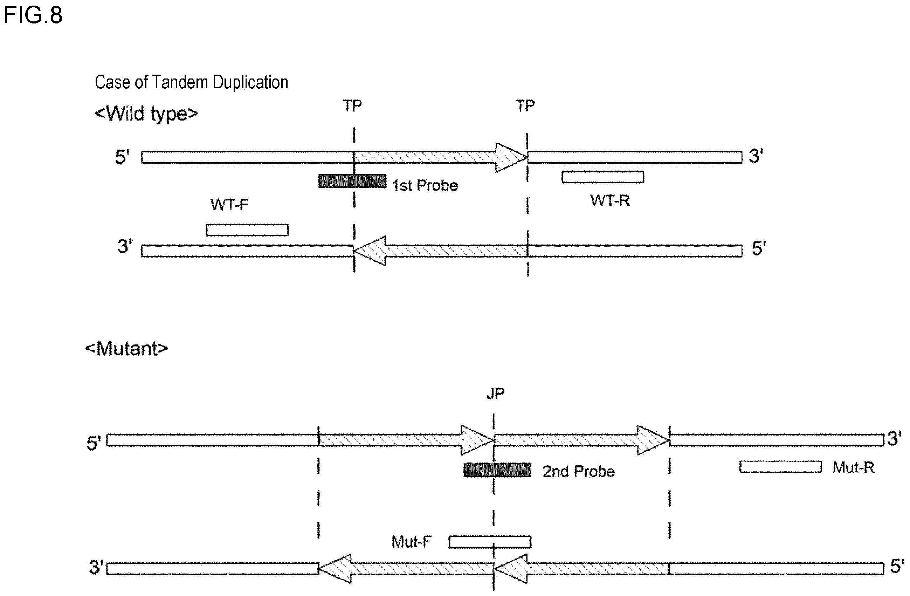

The primer pair set (D) will be described by exemplifying tandem duplication illustrated in Fig. 8. In Fig. 8, for description convenience, a region where tandem duplication occurs is cross-hatched, and each unit of a region repeated by the tandem duplication is illustrated with an arrow.

As illustrated in Fig. 8, the forward primer (WT-F) and the reverse primer (WT-R) of the first primer pair are designed to sandwich a region in "Wild type" corresponding to tandem duplication (region sandwiched between TPs in the drawing) . In this case, the forward primer (WT-F) and the reverse primer (WT-R) of the first primer pair can anneal also to "Mutant", but when these are designed so that the length of the target sequence of the first primer pair when tandem duplication is included may be twice or more than that when tandem duplication is not included, the target sequence of the first primer pair is not amplified when the template is "Mutant", or the amplification efficiency of the target sequence of the first primer pair is conspicuously reduced as compared with that when the template is "Wild type".

On the other hand, the second primer pair is designed so that either of the forward primer (Mut-F) and the reverse primer (Mut-R) may include a portion where tandem duplicated sequences are joined (JP in the drawing), and thus, a region sandwiched between the forward primer (Mut-F) and the reverse primer (Mut-R) is present only when tandem duplication is included. Fig. 8 illustrates an exemplified case where the forward primer (Mut-F) is designed to include the portion where tandem duplicated sequences are joined (JP in Fig. 8), and the other reverse primer (Mut-R) is designed to anneal to a region where tandem duplication does not occur. Owing to this design, only when the template is "Mutant", the target sequence of the second primer pair can be amplified.

When the primer pair set (D) including the first primer pair and the second primer pair described above is used, both a case where tandem duplication of the structural variation occurs and a case where tandem duplication does not occur can be detected by the PCR at one time.

Here, in the exemplified case of Fig. 8, the reverse primer (WT-R) of the first primer pair and the reverse primer (Mut-R) of the second primer pair may be common primers having the same nucleotide sequence.

### [Detection of Presence/absence of Specific Structural Variation based on PCR]

The detection step of the present embodiment includes a detection step of detecting the presence/absence of the specific structural variation by performing PCR using, as a template, a sample DNA, and using, as a primer, a primer pair set including a first primer pair and a second primer pair. As a method to be employed in the detection of the presence/absence of the specific structural variation, any of various methods can be employed as long as amplification of a target sequence by the first primer pair and amplification of a target sequence by the second primer pair can be distinguishably detected. Examples include a method for detecting, by a DNA microarray method, each amplification product obtained by PCR, and a method for analyzing a nucleotide sequence of each amplification product with a next generation sequencer. The DNA microarray method is a method in which DNA fragments are disposed on a substrate at a high density, and DNA sequences on the substrate are hybridized to analyze gene information.

In one embodiment, the PCR performed in the detection step is preferably PCR capable of quantitative determination of an amplification product (hereinafter, simply referred to as quantitative PCR) . In this case, the amount of the amplification product can be detected/quantitatively determined, and therefore, the presence/absence of the specific structural variation can be detected by analyzing the result of the PCR without analyzing, for example, the amplification product with a sequencer or the like. Now, a method for detecting the presence/absence of the specific structural variation employed when the PCR performed in the detection step is quantitative PCR will be described.

### (Detection by Quantitative PCR)

In the quantitative PCR, the amount of an amplification product can be quantitatively determined by a method in which a nucleic acid probe binding to a target sequence and having a labeling function (hereinafter, simply referred to as the probe) or an intercalating dye is used to detect the amount as a light signal. From the viewpoint of more specifically detecting the amplification product, however, quantitative determination of an amplification product is performed in the quantitative PCR preferably using a nucleic acid probe that binds to a target sequence and has a labeling function, and the quantitative PCR may be real-time PCR or digital PCR.

In the present invention, a nucleic acid probe (or simply a probe) refers to a nucleic acid capable of binding (hybridizing) to a target nucleic acid having a complementary sequence. Accordingly, a nucleic acid probe may be lack of complete complementarity to a target nucleic acid as long as it can bind to a target nucleic acid having a complementary sequence . Besides, a nucleic acid probe having a labeling function is preferably a fluorescent probe. A fluorescent probe is hydrolyzed by DNA polymerase between amplification cycles, and a quenching portion is thus removed to emit fluorescence.

A nucleic acid probe used in the quantitative PCR of the present embodiment includes one or more nucleic acid probe sets each consisting of a first probe that hybridizes to the target sequence of the first primer pair, and a second probe that hybridizes to the target sequence of the second primer pair. Besides, the first probe and the second probe are labeled by different fluorescent labels, and in the quantitative PCR, fluorescence derived from the first probe and fluorescence derived from the second probe are detected, and thus, the amount of amplification of the target sequence of the first primer pair and the amount of amplification of the target sequence of the second primer pair are both quantitatively determined. In this manner, the amount of amplification of the target sequence of the first primer pair and the amount of amplification of the target sequence of the second primer pair can be compared, and hence, the presence/absence of the structural variation can be more simply detected. It is noted that the primer pair and the nucleic acid probe are in a relationship of one-to-one correspondence.

In one embodiment, in the method for analyzing the presence/absence of the specific structural variation, sequence analysis (what is called amplicon sequencing) of the amplification product amplified by the PCR is not performed. This is because the amount of amplification of the target sequence of the first primer pair and the amount of amplification of the target sequence of the second primer pair can be both detected by the PCR, and thus, the presence/absence of the structural variation can be detected, and a ratio between the wild type nucleotide sequence and the mutant nucleotide sequence can be measured. Since the sequencing of the amplification product is not performed, a result of the detection of the presence/absence of the specific structural variation can be more simply obtained, and in addition, cost and time required for the experiment can be reduced.

In one embodiment, the method for analyzing the presence/absence of the specific structural variation may include a calculation step of calculating a ratio between the wild-type nucleotide sequence and the mutant nucleotide sequence by comparing, based on the result of the quantitative determination by the PCR, the amount of amplification of the target sequence of the first primer pair and the amount of amplification of the target sequence of the second primer pair. When this step is included, the ratio between the amount of the wild-type nucleotide sequence and the amount of the mutant nucleotide sequence present in the sample DNA can be grasped, and therefore, for example, the situation of cancer of a cancer patient can be grasped. More specifically, when change in the ratio between the amount of the wild-type nucleotide sequence and the amount of the mutant nucleotide sequence in a subject of a patient is periodically observed by periodically collecting the subject from the patient under or after treatment, and applying this method to a sample DNA obtained from the subject, a treatment situation of the disease of the patient and a recurrence situation of the disease can be grasped.

Besides, it is preferable that the first probe be designed to have at least a part thereof to hybridize to a region, in the target sequence of the first primer pair, having a nucleotide sequence different from the target sequence of the second primer pair, and that the second probe is designed to have at least a part thereof to hybridize to a region, in the target sequence of the second primer pair, having a sequence different from the target sequence of the first primer pair. When the first probe and the second probe are thus designed, the amplification of the target sequence of the first primer pair and the amplification of the target sequence of the second primer pair can be more definitely distinguishably measured by the quantitative PCR, and thus, accurate quantitative determination can be made.

Now, a position where the first probe hybridizes to the target sequence of the first primer pair, and a position where the second probe hybridizes to the target sequence of the second primer pair in the present embodiment will be exemplarily described with reference to Figs. 1 to 8 . When probes designed to hybridize to the following exemplified positions are used, the first probe more specifically hybridizes to the target sequence of the first primer pair, and the second probe more specifically hybridizes to the target sequence of the second primer pair, resulting in improving detection capability for the presence/absence of a structural variation by the PCR. Although each primer is illustrated to hybridize to the sense strand of a template DNA in Figs. 1 to 8, each primer may hybridize to the antisense strand of the template DNA.

### (Nucleic Acid Probe Set (A))

First, with Fig. 1 (case of insertion) taken as an example, a nucleic acid probe set (A) for the primer pair set (A) will be described. The first probe hybridizes to a region upstream (on the 5' end side) from a breakpoint (BP in the drawing) of a nucleotide sequence, in the target sequence of the forward primer (WT-F) and the reverse primer (WT-R) of the first primer pair, corresponding to the structural variation in "Wild type", or hybridizes to a portion including the breakpoint of the nucleotide sequence. In other words, the first probe hybridizes to a sequence of a region, in the target sequence of the first primer pair, not common to the target sequence of the second primer pair, or hybridizes to a portion including the region not common. On the other hand, the second probe hybridizes to a portion, in the target sequence of the forward primer (Mut-F) and the reverse primer (Mut-R) of the second primer pair, including a joining point (JP in the drawing) between an inserted region and another region. Alternatively, the second probe may hybridize to an inserted region in the target sequence of the second primer pair. In other words, the second probe hybridizes to a sequence, in the target sequence of the second primer pair, of a region not common to the target sequence of the first primer pair, or hybridizes to a portion including the region not common. When the first probe and the second probe are thus designed, a case where insertion occurs and a case where it does not occur can be more definitely distinguishably detected by the quantitative PCR.

### (Nucleic Acid Probe Set (B))

Next, with Figs. 2 and 3 (case of translocation) taken as an example, a nucleic acid probe set (B) for the primer pair set (B) will be described. First, in the case illustrated in Fig. 2, the first probe hybridizes to a portion, in the target sequence of the first primer pair, downstream from a breakpoint (BP in the drawing) of a nucleotide sequence, or hybridizes to a region, in the target sequence of the first primer pair, including the breakpoint of the nucleotide sequence. In other words, the first probe hybridizes to a sequence, in the target sequence of the first primer pair, of a region not common to the target sequence of the second primer pair, or hybridizes to a portion including the region not common. On the other hand, the second probe hybridizes to a region, in the target sequence of the second primer pair, corresponding to "Seq. 2" that is replaced by translocation, or hybridizes to a region, in the target sequence of the second primer pair, including a joining point (JP in the drawing) between sequences corresponding to "Seq. 1" and "Seq. 2". In other words, the second probe hybridizes to a sequence, in the target sequence of the second primer pair, of a region not common to the target sequence of the first primer pair, or hybridizes to a portion including the region not common.

In the case of translocation illustrated in Fig. 3, the first probe hybridizes to a region, in the target sequence of the first primer pair, upstream from a breakpoint (BP in the drawing) of a nucleotide sequence, or hybridizes to a region, in the target sequence of the first primer pair, including the breakpoint (BP) of the nucleotide sequence. In other words, the first probe hybridizes to a sequence, in the target sequence of the first primer pair, of a region not common to the target sequence of the second primer pair, or hybridizes to a portion including the region not common. On the other hand, the second probe hybridizes to a region, in the target sequence of the second primer pair, corresponding to "Seq. 2" that is a region replaced by translocation, or hybridizes to a region including a joining point (JP in the drawing) between sequences corresponding to "Seq. 2" and "Seq. 1". In other words, the second probe hybridizes to a sequence, in the target sequence of the second primer pair, of a region not common to the target sequence of the first primer pair, or hybridizes to a portion including the region not common. When the first probe and the second probe are thus designed, a case where translocation occurs and a case where it does not occur can be more definitely distinguishably detected by the PCR.

Next, with Figs. 4 and 5 (case of inversion) taken as an example, the nucleic acid probe set (B) for the primer pair set (B) will be described. As illustrated in Figs. 4 and 5, in the case of inversion, the first probe hybridizes to a region, in the target sequence of the first primer pair, corresponding to inversion in "Wild type", or hybridizes to a portion including the region corresponding to inversion. In other words, the first probe hybridizes to a sequence, in the target sequence of the first primer pair, of a region not common to the target sequence of the second primer pair, or hybridizes to a portion including the region not common. On the other hand, the second probe hybridizes to a region, in the target sequence of the second primer pair, where inversion occurs in "Mutant", or hybridizes to a portion including the region where inversion occurs. In other words, the second probe hybridizes to a sequence, in the target sequence of the second primer pair, of a region not common to the target sequence of the first primer pair, or hybridizes to a portion including the region not common. When the first probe and the second probe are thus designed, a case where inversion occurs and a case where it does not occur can be more definitely distinguishably detected by the PCR.

### (Nucleic Acid Probe Set (C))

Next, with Figs. 6 and 7 (case of deletion) taken as an example, a nucleic acid probe set (C) for the primer pair set (C) will be described. First, in the case of deletion illustrated in Fig. 6, the first probe hybridizes to a region, in the target sequence of the first primer pair, in "Wild type" corresponding to a deleted region in "Mutant" (portion sandwiched between BPs in the drawing), or hybridizes to a portion, in the target sequence of the first primer pair, including a breakpoint (BP) of a nucleotide sequence. In other words, the first probe hybridizes to a sequence, in the target sequence of the first primer pair, of a region not common to the target sequence of the second primer pair, or hybridizes to a portion including the region not common. On the other hand, the second probe hybridizes to a region, in the target sequence of the second primer pair, downstream from a portion where deletion occurs (DP in the drawing), or hybridizes to a portion including the portion where deletion occurs (DP in the drawing) . In other words, the second probe hybridizes to a sequence, in the target sequence of the second primer pair, of a region not common to the target sequence of the first primer pair, or hybridizes to a portion including the region not common.

In the case of deletion illustrated in Fig. 7, the first probe hybridizes to, in the same manner as in Fig. 6, a region, in the target sequence of the first primer pair, of "Wild type" corresponding to a deleted region in "Mutant" (portion sandwiched between BPs in the drawing), or hybridizes to a portion including a breakpoint (BP) of a nucleotide sequence disposed in a region sandwiched between the first primer pair. In other words, the first probe hybridizes to a sequence, in the target sequence of the first primer pair, of a region not common to the target sequence of the second primer pair, or hybridizes to a portion including the region not common. On the other hand, the second probe hybridizes to a region, in the target sequence of the second primer pair, upstream from a portion where deletion occurs (DP in the drawing), or hybridizes to a portion including the portion where deletion occurs (DP) . In other words, the second probe hybridizes to a sequence, in the target sequence of the second primer pair, of a region not common to the target sequence of the first primer pair, or hybridizes to a portion including the region not common. When the first probe and the second probe are thus designed, a case where deletion occurs and a case where it does not occur can be more definitely distinguishably detected by the PCR.

### (Nucleic Acid Probe Set (D))

Next, with Fig. 8 (case of tandem duplication) taken as an example, a nucleic acid probe set (D) for the primer pair set (D) will be described. In the case of tandem duplication, the first probe hybridizes to a region, in the target sequence of the first primer pair, upstream from a region in "Wild type" corresponding to tandem duplication (region sandwiched between TPs in the drawing), or hybridizes to a portion, in the target sequence of the first primer pair, including an upstream boundary out of boundaries between a region corresponding to the tandem duplication and other regions in "Wild type" (TP on the upstream side in the drawing) . In other words, the first probe hybridizes to a sequence, in the target sequence of the first primer pair, of a region not common to the target sequence of the second primer pair, or hybridizes to a portion including the region not common. On the other hand, the second probe hybridizes to a portion, in the target sequence of the second primer pair, including a joining point (JP in the drawing) where sequences having tandem duplication are joined to each other. In other words, the second probe hybridizes to a sequence, in the target sequence of the second primer pair, of a region not common to the target sequence of the first primer pair, or hybridizes to a portion including the region not common. When the first probe and the second probe are thus designed, a case where tandem duplication occurs and a case where it does not occur can be more definitely distinguishably detected by the PCR.

The nucleotide sequence of each probe described above may include a complementary nucleotide sequence of the primer. For example, the nucleotide sequence of the first probe may include a nucleotide sequence complementary to the forward primer (WT-F) or the reverse primer (WT-R) of the first primer pair. Besides, the nucleotide sequence of the second probe may include a nucleotide sequence complementary to the forward primer (Mut-F) or the reverse primer (Mut-R) of the second primer pair. When the probes are thus designed, difficulty in probe preparation can be reduced.

Besides, when two or more primer pair sets and two or more nucleic acid probe sets are used in the quantitative PCR, the primer pair sets and the nucleic acid probe sets are in a relationship of one-to-one correspondence. At this point, fluorescent labels used in the first probe and the second probe of each nucleic acid probe set are preferably different from each other. For example, when a primer pair set (A') and a primer pair set (B') are used in the quantitative PCR, a nucleic acid probe set (A') and a nucleic acid probe set (B') corresponding to these are used. A first probe and a second probe of the nucleic acid probe set (A'), and a first probe and a second probe of the nucleic acid probe set (B') have different fluorescent labels. Here, when two or more primer pair sets and two or more nucleic acid probe sets are used in the quantitative PCR, two more or structural variations can be detected.

### [Monitoring Step]

One embodiment of the present invention may include a monitoring step of obtaining the subject from the same individual over time, and analyzing the present/absence of the specific structural variation in each sample DNA obtained from the subject for monitoring change over time of the state of the specific structural variation of the individual. For example, when a patient having a disease caused by the specific structural variation is treated, a subject is collected every timing of before the treatment, immediately after the treatment, and a prescribed period elapsed after the treatment, and a sample DNA is obtained from the subject. Then, the method according to one embodiment of the present invention is applied to each sample DNA to analyze the presence/absence of the specific structural variation, and thus, change over time of the state of the specific structural variation in the patient is monitored. When the monitoring step is included, the effect of the treatment and the health condition of the patient can be evaluated over time, and usefulness of the method of the present invention is thus improved.

### [Genomic DNA Extraction Step/Free DNA Isolation Step]

In one embodiment of the present invention, the method may include, when the sample DNA includes a genomic DNA or a free DNA, a step of extracting the genomic DNA from the subject, or a step of isolating the free DNA from the subject. Such a step can be performed by a method known to those skilled in the art. A method for extracting a genomic DNA from a subject, and a kit for isolating a free DNA can be purchased for use from, for example, Takara Bio Inc., Qiagen, Thermo Fisher Scientific K.K., Omega Bio-tek, MO BIO Laboratories, Promega Corp., GenScript Biotech Corporation, and the like. When the genomic DNA or the free DNA extracted/isolated through the extraction/isolation step is used in the PCR, a nucleotide sequence of interest can be more efficiently amplified than when the subject is directly used in the PCR or the like, and detection accuracy is thus improved.

### [Whole Genome Sequence Analysis Step for Genomic DNA/Free DNA]

One embodiment of the present invention may include, when the sample DNA derived from the subject includes a genomic DNA or a free DNA, a whole genome sequence analysis step of performing whole genome sequence analysis on the sample DNA. The whole genome sequence analysis step is a step performed before the PCR of the detection step. Here, the whole genome sequence analysis is a method for exhaustively analyzing the nucleotide sequence of the whole genome, and is a technique in which, for example, a next generation sequencer (NGS) and a supercomputer are used to analyze the whole genomic information of an individual or cancer so as to identify differences and change thereof. Besides, the present embodiment may include a determination step of determining the specific structural variation based on a result of the whole genome sequence analysis step. When these steps are included, the structural variation causing the disease of each patient can be more accurately grasped, and treatment accuracy in each patient can be remarkably improved.

In one embodiment of the present invention, a next generation sequencer means a sequencer classified in contrast to a capillary sequencer utilizing Sanger method (designated as a first generation sequencer). The next generation sequencer encompasses second generation, third generation, and fourth generation sequencers, and sequencers developed in the future . A next generation sequencer most popular at present is a sequencer using principle that a nucleotide sequence is determined by capturing fluorescence or light emission in connection with complementary strand synthesis by a DNA polymerase or complementary strand binding by a DNA ligase . Specific examples include MiSeq (Illumina), HiSeq 2000 (Illumina, HiSeq being registered trademark), Roche 454 (Roche), NovaSeq 6000 (Illumina), DNBSEQ-G400 (MGI), and ENBSEQ-T7 (MGI).

Means for aligning sequence data obtained by the next generation sequencer can be Burrows-Wheeler Aligner (BWA), and the sequence data can be mapped in known human genomic sequence by BWA. An example of means for analyzing gene, after removing PCR duplication, incorrect sequence read, and an adaptor sequence with SAMtools or the like, includes an analysis pipeline, such as Mutect2
(https://gatk.broadinstitute.org/hc/en-us/articles/3600375938 51-Mutect2), or Genomon
(https://genomon-project.github.io/GenomonPagesR/) .

### [Complementary DNA Synthesis Step]

In one embodiment of the present invention, the method may include a step of extracting an RNA from the subject for synthesizing a complementary DNA by using the RNA as a template. This step can be performed by a method known to those skilled in the art. For example, for extraction of an RNA, a phenol/chloroform method, an AGPC (acid guanidinium thiocyanate-phenol-chloroform extraction) method, a method using a special magnetic particle coated with silica, a commercially available RNA purification reagent (such as TRIzol(R) Reagent, RNeasy(R), QIAzol(R), or ISOGEN), or the like can be used. Besides, in synthesis of a complementary DNA using an RNA as a template, a reaction solution containing the RNA, a primer, a reverse transcriptase, and a substrate may be incubated to make the reverse transcriptase act on the RNA for synthesis of a cDNA. As the primer, a primer targeting a specific RNA to be analyzed may be used. When the cDNA is used in the PCR, a nucleotide sequence of interest can be more efficiently amplified than when the subject is directly used in the PCR or the like, and detection accuracy is thus improved. Besides, the present embodiment may include a step of sequencing the thus synthesized complementary DNA to determine the specific structural variation based on the result of the sequencing.

### [Amplicon Sequencing Step]

The method of one embodiment of the present invention may include an amplicon sequencing step of sequencing an amplification product (amplicon) obtained by the PCR. In the method of the present embodiment, however, the presence/absence of the specific structural variation can be detected, and the ratio between the wild-type nucleotide sequence and the mutant nucleotide sequence can be measured even when the amplicon sequencing step is not included.

A method for performing the sequencing in the amplicon sequencing step is not especially limited, and is preferably a method using the above-described next generation sequencer. In the sequencing using the next generation sequencer, for example, a primer pair to which an adaptor sequence necessary for the analysis is ligated is used to further amplify the amplification product obtained by the PCR, and thereafter, an optional identification sequence and a sequence for the sequencing are added thereto by index PCR, and thus, the sequencing can be performed with various sequencers. Besides, when an adaptor sequence is added to each primer pair used in the PCR, the amplification product may be used for the sequencing.

### [Minimal Residual Disease (MRD) Analysis]

In one embodiment of the present invention, a method for analyzing the presence/absence of the specific structural variation can be used for analyzing minimal residual disease (MRD) . The minimal residual disease (MRD) refers to a small number of cancer cells remaining in the body during and after treatment, and possibly ultimately causing recurrence of the disease. In the method of the present embodiment, MRD can be indirectly detected by detecting a specific structural variation of the MRD.

For example, when a subject is periodically collected from a patient under or after treatment, and the method of the present embodiment is applied to the subject, the presence/absence and increase/decrease of MRD in the subject of the patient are periodically observed, and thus, a treatment situation of the disease of the patient and a recurrence situation of the disease can be grasped.

### [Primer Pair Set]

One embodiment of the present invention provides a primer pair set including a first primer pair and a second primer pair, to be used for detecting presence/absence of a specific structural variation in a sample DNA containing a genomic DNA, a free DNA, or a complementary DNA derived from a subject, and used for performing am amplification reaction by PCR using the sample DNA as a template. The types of the primer pair set can be the above-described primer pair sets (A) to (D).

The aforementioned embodiments are described for making the present invention easily understood, and are not intended to limit the present invention. Accordingly, the respective elements disclosed in the aforementioned embodiments are intended to encompass all design modifications and equivalents belonging to the technical scope of the present invention.

It is noted that the following embodiments are encompassed in the scope of the present invention: The method according to claim 1 having the characteristics according to claim 2; the method according to claim 1 or 2 having the characteristics according to claim 3; the method according to any one of claims 1 to 3 having the characteristics according to claim 4; the method according to claim 4 having the characteristics according to claim 5; the method according to claim 5 having the characteristics according to claim 6; the method according to any one of claims 4 to 6 having the characteristics according to claim 7; the method according to any one of claims 4 to 7 having the characteristics according to claim 8; the method according to any one of claims 1 to 8 having the characteristics according to claim 9; the method according to any one of claims 1 to 9 having the characteristics according to claim 10; the method according to any one of claims 1 to 10 having the characteristics according to claim 11; the method according to any one of claims 1 to 11 having the characteristics according to claim 12; the method according to any one of claims 1 to 12 having the characteristics according to claim 13; the method according to any one of claims 1 to 13 having the characteristics according to claim 14; the MRD analysis method according to claim 15 having the characteristics according to claims 1 to 14; a primer pair set having the characteristics according to claim 16; and a method for designing a primer pair set having the characteristics according to claim 17.

### EXAMPLES

The present invention will now be specifically described with reference to the following examples, but the present invention is not limited by these examples . It is noted that informed consent was obtained from all patients for subjects used in experiments.

### <Example 1: Detection of Presence/absence of Translocation>

### (1) Obtaining of Subject and Extraction of Genomic DNA

### (Obtaining of Subject)

The bone marrow was obtained as a subject from an acute lymphoblastic leukemia (ALL) patient (hereinafter referred to as the patient A). Besides, an oral mucosa cell was collected with a swab from the same patient A as a normal tissue comparison sample (control).

### (Extraction of Genomic DNA)

From the bone marrow, a genomic DNA was extracted with Gentra Puregene Blood kit (manufactured by Qiagen). Besides, a genomic DNA was extracted from the oral mucosa cell with QIAamp DNA Mini kit (manufactured by Qiagen).

### (2) Whole Genome Sequence Analysis, and Determination of Specific Structural Variation to be Detected

### (Sequencing with Next Generation Sequencer)

Whole genome sequence analysis was performed by using the extracted genomic DNA. NGS library preparation was performed with Truseq DNA Nano Library Prep kit (manufactured by Illumina). A library was created through procedures of performing, on 100 ng of DNA physically fragmented with Covaris M220 (Covaris), end-joining repair, size selection, adaptor addition, PCR amplification, and concentration. The thus created library was quantitatively determined with Qubit dsDNA HS Assay kit (Thermo Fisher Scientific), and was confirmed that a single band was amplified through electrophoresis with Agilent 4200 Tapestation (Agilent). The created library was sequenced with Novaseq 6000 (Illumina) by Macrogen Japan to obtain a FASTQ file including read information.

### (Data Analysis using Supercomputer)

The FASTQ file obtained by the above-described sequencing was submitted, for analysis, to a supercomputer SHIROKANE 4 of Human Genome Center, the Institute of Medical Science, the University of Tokyo. The data analysis was conducted using pipeline software Genomon 2 (https://genomon.readthedocs.io/ja/latest/) exclusive for next generation sequencing data. After conducting preprocessing of removing, from the sequencing data, an adaptor sequence, an index sequence inherent to the sample, a duplicated read caused in PCR, and a read of low sequence quality, mapping was conducted with Genome Reference Consortium Human Build 37 (GRCh37, hg19) used as a reference sequence. A sequence read of location information having a mutation as compared with the mapped reference sequence was subjected to calculation processing using a statistical test, and the DNA derived from the bone marrow sample and the control (DNA derived from the oral mucosa cell) were compared with each other to detect location information in which a mutation was significantly observed. The statistical test was performed by Fisher's exact test, and a rejection region of null hypothesis was set with a p value < 0.10. Single nucleotide variants (SNVs), nucleotide insertion/deletion (Indels), and fusion gene abnormality (Fusion) were detected, and annotation was conducted based on database of ANNOVAR
(https://annovar.openbioinformatics.org/en/latest/) .

### (Determination of Specific Structural Variation to be Detected)

Subsequently, Genomon 2 was used to specify a cancer-related gene mutation that could be involved in onset of ALL among gene mutations detected from data resulting from the whole genome sequence analysis, namely, a structural variation that could be a driver mutation of the disease. First, a mutation having a ratio of allele with gene mutation (variant allele frequency: VAF) of less than 3%, known non-pathological mutations registered in a plurality of single nucleotide polymorphism database including NCBI dbSNP (https://www.ncbi.nlm.nih.gov/snp/), a mutation that was present also in the control sample (derived from the oral mucosa cell) and was probably a sequencing error, a mutation having low sequence read quality, and a mutation not involving amino acid substitution were eliminated. Next, referring to tumor database such as COSMIC version 90, 91 (https://cancer.sanger.ac.uk/cosmic), or the Guideline for Genetic Testing in Hematopoietic Tumor 2018, 2020 from Japanese Society of Hematology
(http://www.jshem.or.jp/genomgl/home.html), registered mutations, and deleterious mutations such as insertion and deletion of nucleotides in hematopoietic tumor-related genes were extracted to determine whether or not these were driver mutations. A mapped read was visually confirmed using Integrative Genomics Viewer version 2.8.2
(https://software.broadinstitute.org/software/igv/), and a breakpoint due to a structural variation was also identified.

Although it is assumed that the identification of the driver gene mutation is conducted by comparing the gene mutation of the subject with the gene mutation of the oral mucosa sample used as the normal control, it was also necessary to consider the influence of CHIP (clonal hematopoiesis with indeterminate potential) mutation, and it was also confirmed that there was no discrepancy between the detected variant allele frequency and a ratio of cancerous cells obtained by a clinical test.

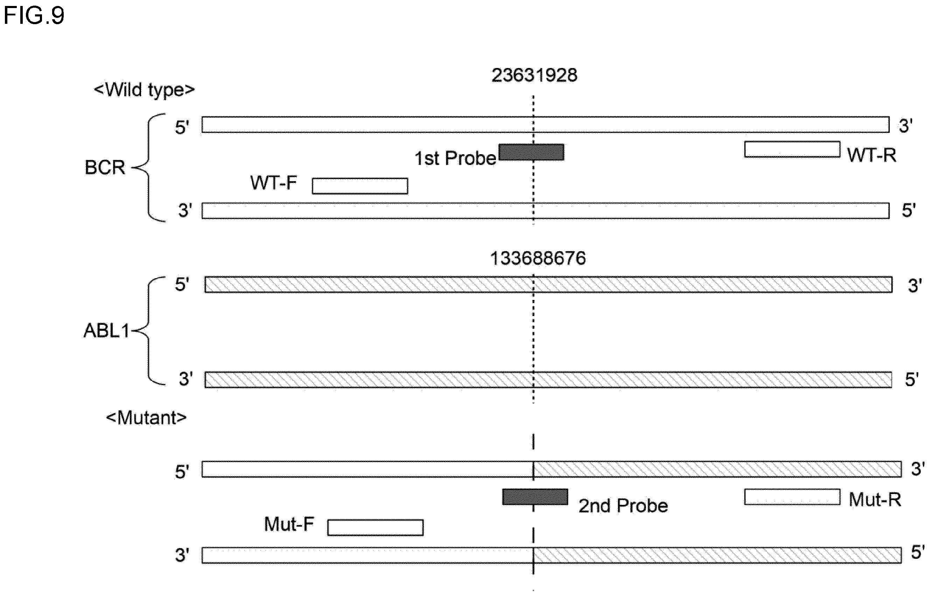

As a result of the above-described analysis, BCR-ABL fusion gene having translocation occurring between a nucleotide at position 23631928 in the BCR gene on chromosome 22 and a nucleotide at position 133688676 in the ABL gene on chromosome 9 was discovered as the driver mutation probably involved in the onset of ALL in the patient A (see Fig. 9). Therefore, the BCR-ABL fusion gene was determined as the specific structural variation to be detected.

### (3) Detection of Structural Variation by Quantitative PCR

### (Creation of Primer and Probe)

A first primer pair, a second primer pair, a first probe and a second probe to be used for detecting the presence/absence of the BCR-ABL fusion gene determined above as the specific structural variation of the patient A were created. The created primers and probes are shown in Table 1. It is noted that SEQ ID NO: 1 of the sequence listing corresponds to the forward primer of the first primer pair, SEQ ID NO: 2 corresponds to the reverse primer of the first primer pair, SEQ ID NO: 3 corresponds to the forward primer of the second primer pair, SEQ ID NO: 4 corresponds to the reverse primer of the second primer pair, SEQ ID NO: 5 corresponds to the first probe, and SEQ ID NO: 6 corresponds to the second probe.

**[Table 1]**

| | | Nucleotide sequence (5' to 3') |
|---|---|---|
| First primer pair | Forward (SEQ ID NO: 1) | TTTTGATGGGACTAGTGGACTTTG |
| | Reverse (SEQ ID NO: 2) | AACTGCTCCTAACCCACCTTGTC |
| Second primer pair | Forward (SEQ ID NO: 3) | TTTTGATGGGACTAGTGGACTTTG |
| | Reverse (SEQ ID NO: 4) | TCTTGATCTCCTGACCTCGTGAT |
| First probe (SEQ ID NO: 5) | | CAGAAGGAAGAGCTATGC |
| Second probe (SEQ ID NO: 6) | | CAGAAGGCGGTGGCT |

As illustrated in Fig. 9, the first primer pair was designed to sandwich a portion in the BCR corresponding to the specific structural variation, namely, a breakpoint (23631928 in the drawing) at which the nucleotide sequence of the BCR is broken on the occurrence of translocation. On the other hand, the second primer pair was designed to allow the forward primer (Mut-F) to anneal to a sequence corresponding to the BCR in "Mutant", and the reverse primer (Mut-F) to anneal to a sequence corresponding to the ABL1. The first probe was designed to hybridize to a region sandwiched by the first primer pair, and including the breakpoint (23631928 in the drawing) of the nucleotide sequence. On the other hand, the second probe was designed to hybridize to a region sandwiched by the second primer pair, and including a joining point of the sequences corresponding to the BCR and the ABL1. Besides, the forward primers of the first primer pair and the second primer pair were created as common primers having the same sequence.

The sequences of the primers and the probes were designed with Primer Express version 5.0 (Thermo Fisher Scientific). Besides, the probes were modified with Black Hole Quencher (BHQ, registered trademark) 1 as a quencher at the 3' end, and as a fluorescent dye, the second probe was modified with 6-carboxyfluorescein (FAM) and the first probe was modified with 6-carboxy-2,4,4,5,7,7-hexchlorofluorescein succinimidyl ester (HEX) at the 5' end. The synthesis of the primers and the probes were conducted by FASMAC.

### (Digital PCR and Analysis of Results thereof)

Subsequently, the DNA derived from the bone marrow sample obtained by the DNA extraction described above and the DNA derived from the oral mucosa cell used as the control were subjected to digital PCR.

First, to each DNA, ddPCR Supermix (Bio-Rad) was added, and the first primer pair, the second primer pair, the first probe, and the second probe created as described above were further added thereto to obtain a prepared sample.

Subsequently, Droplet Generator Oil for Probes (Bio-Rad) and the prepared sample were dispensed into an exclusive cartridge of QX200 Droplet Generator (Bio-Rad), and the resultant cartridge was set in the generator to prepare a droplet.

Next, DNA included in the prepared droplet was amplified by performing PCR with Veriti Dx thermal cycler (Thermo Fisher Scientific). After initial denaturation at 95°C for 10 minutes, annealing at 94°C for 10 seconds and at 58 to 61°C for 1 minute was performed for 40 cycles, and extension reaction was performed at 98°C for 10 minutes.

### (Analysis of Digital PCR Results)

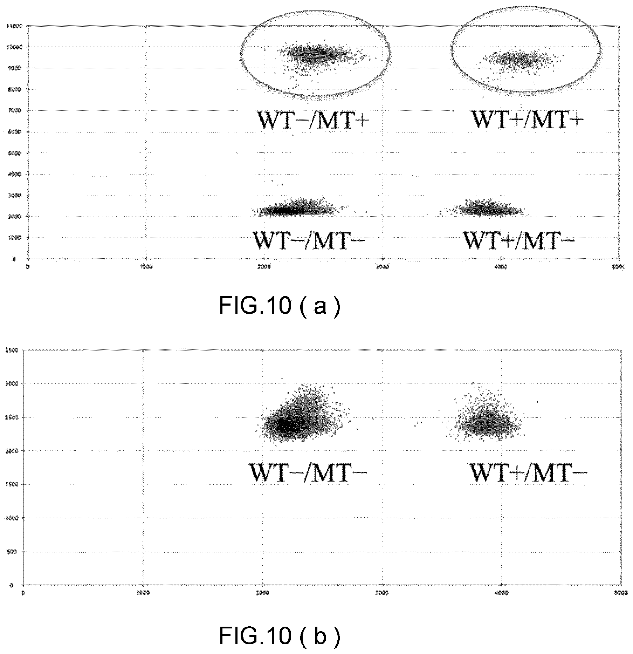

By measuring fluorescence of the droplet with QX200 Droplet Reader and QuantaSoft (both Bio-Rad), and analyzing the result, it was confirmed whether or not the presence/absence of translocation, that is, the specific structural variation of the patient A, could be detected. The results are illustrated in Fig. 10.

Fig. 10(a) illustrates the analysis result of the bone marrow-derived sample DNA, and Fig. 10 (b) illustrates the analysis result of the sample DNA derived from the oral mucosa cell used as the normal control. In these drawings, "WT" means the wild type (namely, the sample in which the structural variation does not occur), "MT" means the mutant (namely, the sample in which the structural variation occurs), "+ (plus)" indicates a result that the nucleotide sequence was amplified and detected, and "- (minus) " indicates a result that the nucleotide sequence was not amplified and not detected.

For example, "WT-/MT+" in the drawing indicates a result that a droplet in which the wild-type nucleotide sequence was not amplified but that of the mutant alone was amplified was detected. In other words, "WT-/MT+" indicates a result that the presence of the specific structural variation was detected. Besides, "WT+/MT-" in the drawing indicates a result that a droplet in which the wild-type nucleotide sequence was amplified but the mutant nucleotide sequence was not amplified was detected. In other words, "WT+/MT-" indicates a result that the absence of the specific structural variation was detected. Besides, "WT+/MT+" in the drawing indicates a result that a droplet in which both the wild-type nucleotide sequence and the mutant nucleotide sequence were amplified was detected. "WT-/MT-" in the drawing indicates a result that a droplet in which neither of the wild-type nucleotide sequence and the mutant nucleotide sequence was amplified was detected.

As illustrated in Fig. 10 (a), as a result of the digital PCR using the bone marrow-derived DNA as a template, WT-/MT+ and WT+/MT+ were confirmed. Specifically, it was confirmed that the mutant nucleotide sequence having translocation, that is, the specific structural variation of the patient A, can be detected in accordance with the method of Example 1. Besides, WT+/MT- was also detected in a sample DNA having deletion obtained from the patient-derived bone marrow. This reveals that a wild-type nucleotide sequence not having translocation can be detected by the method of Example 1.

On the other hand, as illustrated in Fig. 10 (b), as a result of the digital PCR by using, as a template, the sample DNA derived from the oral mucosa cell used as the normal control, WT-/MT+ and WT+/MT+ were not detected, but WT+/MT- and WT-/MT- were detected. This means that the mutant nucleotide sequence was not detected, but a wild-type nucleotide sequence not having translocation was detected in the sample DNA of the normal control. It was revealed, based on the results illustrated in Figs. 10 (a) and 10 (d) described above, that the presence/absence of the mutant nucleotide sequence having translocation and the presence/absence of a wild-type nucleotide sequence not having translocation in a sample DNA can be simultaneously distinguishably detected by the method of Example 1.

### (Monitoring of Specific Structural Variation)

Example 1 described above provides the method optimized for the patient A for detecting the presence/absence of the BCR-ABL fusion gene, that is, the specific structural variation of the patient A. When, for example, such a method optimized for a specific patient is performed on the specific patient a plurality of times over time, the presence/absence and the increase/decrease of nucleotide sequences having the structural variation in the specificpatient can be monitored. Thus, progression of the disease, treatment effect and the like in the specific patient can be grasped.

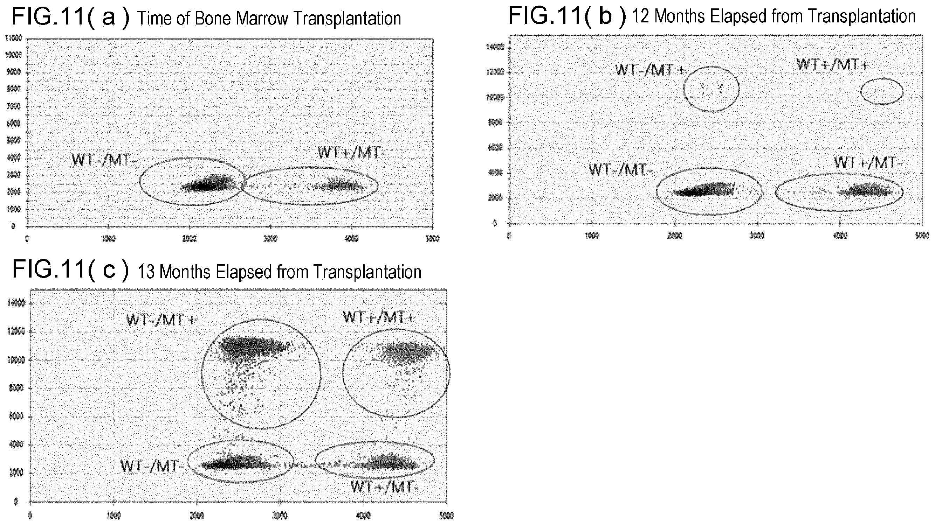

For example, Fig. 11 illustrates results of performing translocation detection, by a method similar to that of Example 1, a plurality of times over time in an ALL patient B having the BCR-ABL fusion gene that is a structural variation similar to that of the patient A. Respective primer pair sets and probes used in digital PCR are shown in Table 2. SEQ ID NO: 7 of the sequence listing corresponds to the forward primer of the first primer pair, SEQ ID NO: 8 corresponds to the reverse primer of the first primer pair, SEQ ID NO: 9 corresponds to the forward primer of the second primer pair, SEQ ID NO: 10 corresponds to the reverse primer of the second primer pair, SEQ ID NO: 11 corresponds to the first probe, and SEQ ID NO: 12 corresponds to the second probe. It is noted that a driver mutation involved in the onset of ALL (BCR-ABL fusion gene) in the patient B is caused by translocation occurring between a nucleotide at position 23632322 in the BCR gene on chromosome 22 and a nucleotide at position 133601463 in the ABL gene on chromosome 9.

**[Table 2]**

| | | Nucleotide sequence (5' to 3') |
|---|---|---|
| First primer pair | Forward (SEQ ID NO: 7) | AGTCTTGCTATGGTGTCTAAGCTGG |
| | Reverse (SEQ ID NO: 8) | CGCAAGGGAGCTCAATCCT |
| Second primer pair | Forward (SEQ ID NO: 9) | AGTCTTGCTATGGTGTCTAAGCTGG |
| | Reverse (SEQ ID NO: 10) | GGGCCAAAAACATACTCATCATG |
| First probe (SEQ ID NO: 11) | | ATACTCCTGCCTCAACCTCCCAAAGTGTTAAGATTA |
| Second probe (SEQ ID NO: 12) | | CTTGAACTCTTGTGAAACCAACTGGATCCTGA |

Fig. 11(a) illustrates a result obtained by analyzing, by a method similar to that of Example 1, the bone marrow collected from the patient B immediately after hematopoietic stem cell transplantation. Fig. 11(b) illustrates a result obtained by analyzing, by the method similar to that of Example 1, the bone marrow collected from the patient B after 12 months elapsed from the treatment of bone marrow transplantation. Besides, Fig. 11(c) illustrates a result obtained by analyzing, by the method similar to that of Example 1, the bone marrow collected from the patient B after 13 months elapsed from the treatment of bone marrow transplantation. As illustrated in Fig. 11 (a), since only WT+/MT- and WT-/MT- were detected immediately after the bone marrow transplantation, the effect of the bone marrow transplantation on the patient B can be confirmed. As illustrated in Fig. 11(b), however, WT-/MT+ and WT+/MT+ started to be found although in a small amount after 12 months, and after 13 months, WT-/MT+ and WT+/MT+ started to be found in a large amount as illustrated in Fig. 11(c). This means that recurrence of ALL in the patient B. In this manner, when the method of Example 1 is performed on the patient B a plurality of times over time, the presence/absence and the increase/decrease of the specific structural variation in the patient B can be monitored, and as a result, the progression of the disease, the treatment effect and the like in the patient B can be grasped.

### <Example 2: Detection of Presence/absence of Deletion>

### (1) Obtaining of Subject and Extraction of Genomic DNA

### (Obtaining of Subject)

The bone marrow was obtained as a subject from an acute myeloid leukemia (AML) patient (hereinafter referred to as the patient C) . Besides, an oral mucosa cell was collected with a swab from the same patient C as a normal tissue comparison sample (normal control).

### (Extraction of Genomic DNA)

Genomic DNAs were extracted from the bone marrow and the oral mucosa cell derived from the patient C by a method similar to that of Example 1.

### (2) Whole Genome Sequence Analysis, and Determination of Specific Structural Variation to be Detected

### (Sequencing with Next Generation Sequencer)

The extracted genomic DNA was used to perform sequencing with a next generation sequencer by a method similar to that of Example 1, and thus a FASTQ file was obtained.

### (Data Analysis using Supercomputer)

By a method similar to that of Example 1, the FASTQ file obtained by the sequencing was submitted, for analysis, to a supercomputer SHIROKANE 4 of Human Genome Center, the Institute of Medical Science, the University of Tokyo.

### (Determination of Specific Structural Variation to be Detected)

By a method similar to that of Example 1, Genomon 2 was used to specify a cancer-related gene mutation that could be involved in onset of AML among gene mutations detected from data resulting from the whole genome sequence analysis, namely, structural variation that could be a driver mutation of the disease . Besides, by a method similar to that of Example 1, a breakpoint due to the structural variation was identified, and it was also confirmed that there was no discrepancy between the detected variant allele frequency and a ratio of cancerous cells obtained by a clinical test.

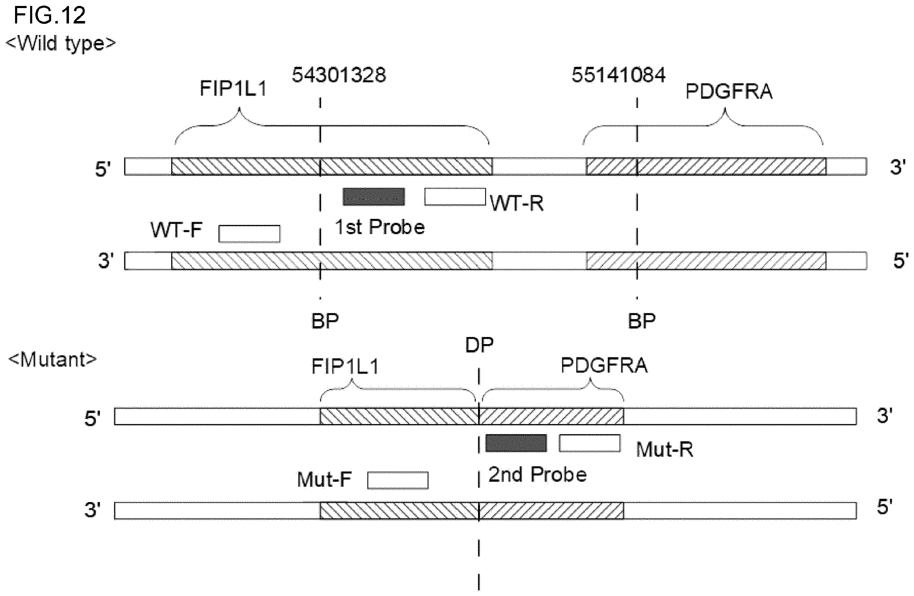

As a result of the above-described analysis, FIP1L1-PDGFRA fusion gene in which a nucleotide sequence between a nucleotide at position 54301328 in the FIP1L1 gene on chromosome 4 and a nucleotide at position 5514084 in the PDGFRA gene on chromosome 4 was deleted (partial deletion of chromosome 4, q12, del (4) (q12), see "Mutant" illustrated in Fig. 12) was discovered as the driver mutation probably involved in the onset of AML in the patient C. Therefore, the FIP1L1-PDGFRA fusion gene was determined as deletion to be detected.

### (3) Detection of Structural Variation by Quantitative PCR

### (Creation of Primer and Probe)

A first primer pair, a second primer pair, a first probe and a second probe to be used for detecting the deletion determined above as the specific structural variation of the patient C (FIP1L1-PDGFRA fusion gene) were created. The created primers and probes are shown in Table 3. It is noted that SEQ ID NO: 13 of the sequencing list corresponds to the forward primer of the first primer pair, SEQ ID NO: 14 corresponds to the reverse primer of the first primer pair, SEQ ID NO: 15 corresponds to the forward primer of the second primer pair, SEQ ID NO: 16 corresponds to the reverse primer of the second primer pair, SEQ ID NO: 17 corresponds to the first probe, and SEQ ID NO: 18 corresponds to the second probe.

**[Table 3]**

| | | Nucleotide sequence (5' to 3') |
|---|---|---|
| First primer pair | Forward (SEQ ID NO: 13) | TCAATTTAAAAAGCCTGCCTATCA |
| | Reverse (SEQ ID NO: 14) | AAAGGGTGCATCCTGGAGG |
| Second primer pair | Forward (SEQ ID NO: 15) | TCAATTTAAAAAGCCTGCCTATCA |
| | Reverse (SEQ ID NO: 16) | CCATGGAACTTACCAAGCACTAGTC |
| First probe (SEQ ID NO: 17) | | AAAGCTTTCTTAGGATGTTGATTTTTGAATGTGCTTT |
| Second probe (SEQ ID NO: 18) | | TGCCTTATGACTCAAGATGGGAGTTTCCAAG |

As illustrated in Fig. 12, the first primer pair was designed to allow the forward primer (WT-F) to anneal to a region of the FIP1L1 not missing due to the deletion, and the reverse primer (WT-R) to anneal to a region of the FIP1L1 missing due to the deletion. On the other hand, the second primer pair was designed to allow the forward primer (Mut-F) to anneal to a region of the FIP1L1 not missing due to the deletion, and the reverse primer (Mut-R) to anneal also to a region of the PDGFRA not missing due to the deletion. At this point, the second primer pair can anneal to a nucleotide sequence not having the deletion, but was designed so that the length of a nucleotide sequence sandwiched between the forward primer (Mut-F) and the reverse primer (Mut-R) when the deletion occurred might be 1/10 or less than that when the deletion did not occur. Besides, the forward primers of the first primer pair and the second primer pair were created as common primers having the same sequence.

Besides, as illustrated in Fig. 12, the first probe was designed to hybridize to a region, in the target sequence of the first primer pair, missing due to the deletion (region sandwiched between BPs in the drawing) . On the other hand, the second probe was designed to hybridize to a region, in the target sequence of the second primer pair, not missing due to the deletion in the PDGFRA.

Respective sequences of the primers and the probes were designed with Primer Express version 5.0 in the same manner as in Example 1, and were synthesized by FASMAC. Besides, also the probes were, in the same manner as in Example 1, modified with BHQ(R) 1 as a quencher at the 3' end, and the second probe was modified with FAM and the first probe was modified with HEX at the 5' end.

### (Digital PCR and Analysis of Results thereof)

Subsequently, the bone marrow sample-derived DNA and the oral mucosa cell-derived DNA used as the control obtained by the above-described DNA extraction were used to perform digital PCR by a method similar to that of Example 1.

### (Analysis and Monitoring of Results of Digital PCR)

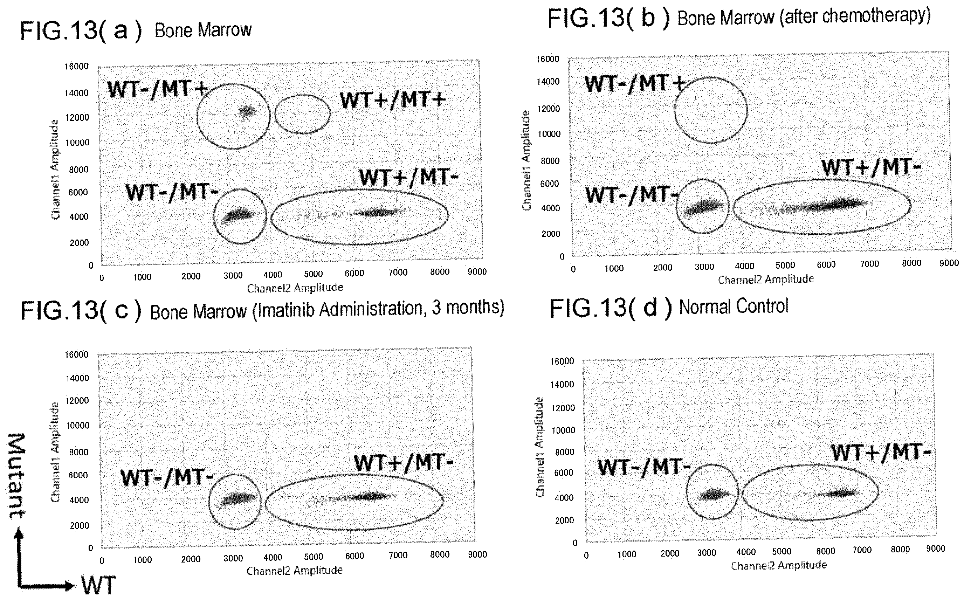

Subsequently, the results of the digital PCR were analyzed by a method similar to that of Example 1. The results are illustrated in Fig. 13.

Fig. 13(a) illustrates analysis results of the sample DNA having deletion obtained from the patient-derived bone marrow. As illustrated in Fig. 13 (a), WT-/MT+ was detected from the sample DNA obtained from the patient-derived bone marrow. This indicates that the mutant nucleotide sequence having deletion can be detected by the method of Example 2. Besides, WT+/MT- was also detected in the sample DNA having deletion obtained from the patient-derived bone marrow. This indicates that a wild-type nucleotide sequence not having deletion can be detected by the method of Example 2.

Fig. 13(d) illustrates analysis results of the oral mucosa cell-derived sample DNA used as the normal control not having deletion. In the sample DNA obtained from the normal control, WT-/MT+ was not detected, but WT+/MT- was detected. This means that a mutant nucleotide sequence was not detected but a wild-type nucleotide sequence not having deletion was detected in the sample DNA of the normal control. It was revealed, based on the results of Figs. 13 (a) and 13 (d), that the presence/absence of the mutant nucleotide sequence having deletion and the presence/absence of the wild-type nucleotide sequence not having deletion in the sample DNA can be simultaneously distinguishably detected by the method of Example 2.

Fig. 13(b) illustrates results of analyzing, by the method of Example 2, the bone marrow obtained again from the patient C after subjecting, to chemotherapy, the patient C to which the method of Example 2 had been applied. It was confirmed that, as compared with the results of Fig. 13 (a), the number of detection of WT-/MT+ was reduced, and the number of detection of WT+/MT- was increased in Fig. 13 (b) . This means that the mutant nucleotide sequence in the bone marrow of the patient C was reduced by the chemotherapy. Besides, Fig. 13 (c) illustrates results of analyzing, by the method of Example 2, the bone marrow collected again from the patient C after 3 months elapsed from the bone marrow collection of Fig. 13 (a), and after administering imatinib, that is, a tyrosine kinase inhibitor, to the patient C periodically for 3 months. In Fig. 13(c), WT-/MT+ was not detected but WT+/MT- alone was detected. This means that the mutant nucleotide sequence in the bone marrow of the patient C was eliminated, or reduced to a level undetectable at least by the method of Example 2 owing to the administration of imatinib. In this manner, when the method of Example 2 was applied a plurality of times over time to the sample DNAs derived from the subjects obtained from the same patient C, the presence/absence and the amount change of the mutant nucleotide sequence and the wild-type nucleotide sequence in the sample DNAs can be monitored. Besides, it was revealed that change in the ratio between the mutant nucleotide sequence and the wild-type nucleotide sequence can be monitored.

### <Example 3: Detection of Presence/absence of Inversion>

### (Obtaining of Subject)

The bone marrow was collected over time from a patient having developed acute myeloid leukemia (AML) due to a driver mutation of inversion occurring between the GATA2 gene on 3q21.3 and the MECOM gene on 3q26.2 (hereinafter referred to as the patient D) . The bone marrow was collected 7 times in total, that is, 17 months before the diagnosis, 15 months before the diagnosis, 10 months before the diagnosis, 9 months before the diagnosis, 4 months before the diagnosis, at the time of the diagnosis, and 5 months after the diagnosis.

### (Extraction of Genomic DNA)

By a method similar to that of Example 1, a sample DNA was collected from each of the bone marrows obtained as described above over time.

### [Detection of Structural Variation by Quantitative PCR]

### (Creation of Primer and Probe)

A first primer pair, a second primer pair, a first probe and a second probe to be used for detecting the inversion occurring between the GATA2 gene on 3q21.3 and the MECOM gene on 3q26.2, that is, the specific structural variation of the patient C, were created. The created primers and probes are shown in Table 4. It is noted that SEQ ID NO: 19 of the sequence listing corresponds to the forward primer of the first primer pair, SEQ ID NO: 20 corresponds to the reverse primer of the first primer pair, SEQ ID NO: 21 corresponds to the forward primer of the second primer pair, SEQ ID NO: 22 corresponds to the reverse primer of the second primer pair, SEQ ID NO: 23 corresponds to the first probe, and SEQ ID NO: 24 corresponds to the second probe.

**[Table 4]**

| | | Nucleotide sequence (5' to 3') |
|---|---|---|
| First primer pair | Forward (SEQ ID NO: 19) | AAATGAGATCAATCAGTACACGATATCTG |
| | Reverse (SEQ ID NO: 20) | CAGGCATGAGCCACTGTGC |
| Second primer pair | Forward (SEQ ID NO: 21) | AAATGAGATCAATCAGTACACGATATCTG |
| | Reverse (SEQ ID NO: 22) | CCTCCTTATTAGAATCTGTACTTCACTGTATT |
| First probe (SEQ ID NO: 23) | | TTTAGTGGGATTGTTTGTTGTGCATTCTGTTCC |
| Second probe (SEQ ID NO: 24) | | TTTAGTGGGATTGTTTGTAAAGCTGGGCG |

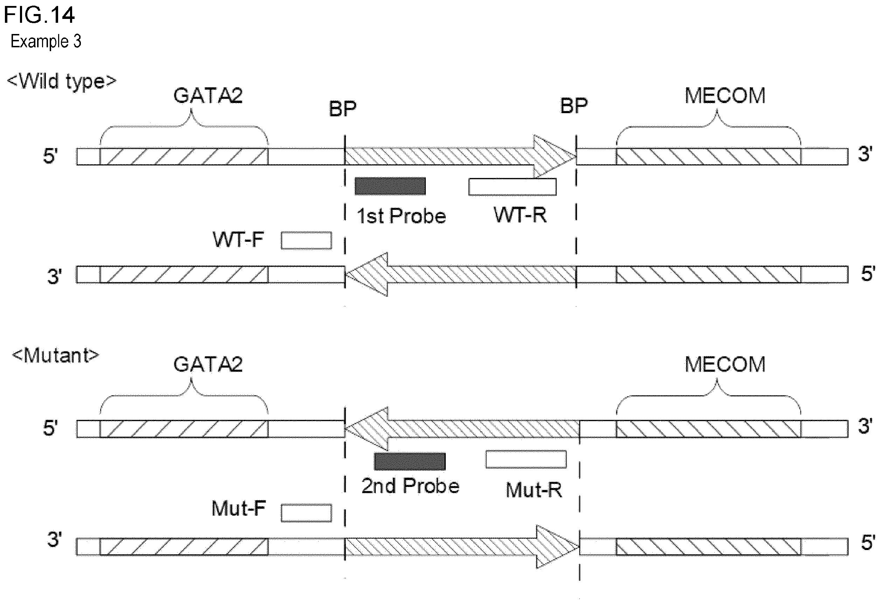

As illustrated in Fig. 14, the first primer pair was designed to allow the reverse primer (WT-R) to anneal to a region in "Wild type" corresponding to a region where the inversion occurred (region sandwiched between BPs in the drawing), and the forward primer (WT-F) to anneal to a region where the inversion did not occur. On the other hand, the second primer pair was designed to allow the reverse primer (Mut-R) to anneal to a nucleotide sequence in "Mutant" where the inversion occurred, and the forward primer (Mut-F) to anneal to a region where the inversion did not occur.

Besides, as illustrated in Fig. 14, the first probe was designed to hybridize to a region, in the target sequence of the first primer pair, of "Wild type" corresponding to the region where the inversion occurred (region sandwiched between BPs in the drawing). On the other hand, the second probe was designed to hybridize to a region, in the target sequence of the second primer pair, where the inversion occurred.

Respective sequences of the primers and the probes were designed with Primer Express version 5.0 in the same manner as in Example 1, and were synthesized by FASMAC. Besides, also the probes were, in the same manner as in Example 1, modified with BHQ(R) 1 as a quencher at the 3' end, and the second probe was modified with FAM and the first probe was modified with HEX at the 5' end.

### (Digital PCR and Analysis of Results thereof)

Subsequently, in accordance with a method similar to that of Example 1, each sample DNA obtained by the genomic DNA extraction described above was subjected to digital PCR using the primer pairs and the probes shown in Table 4.

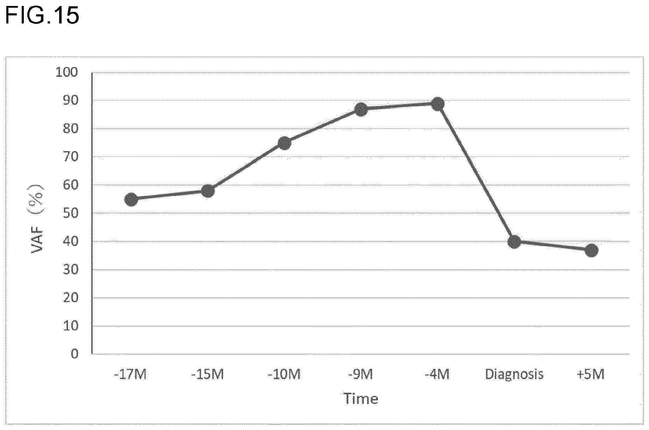

Subsequently, results of the digital PCR were analyzed. Fig. 15 is a graph having the abscissa indicating the chronological order in collecting the bone marrow, and the ordinate indicating the variant allele frequency (VAF (%)). The VAF of each sample DNA is a value obtained by dividing the total number of droplets of WT-/MT+ and WT+/MT+ by the total number of droplets of WT-/MT+, WT+/MT+, and WT+/MT- (VAF = {(number of droplets of WT-/MT+) + (number of droplets of WT+/MT+) } / {(number of droplets of WT-/MT+) + (number of droplets of WT+/MT+) + (number of droplets of WT+/MT-) 1) . Based on the results illustrated in Fig. 15, change over time of the VAF in the patient D could be confirmed.

It was revealed, based on these results, that when the method according to one embodiment of the present invention is applied to each of the sample DNAs derived from a plurality of subjects (bone marrow) obtained from the same patient over time, change in the ratio between the mutant nucleotide sequence and the wild-type nucleotide sequence in the patient can be monitored.

### <Example 4: Detection 2 of Presence/absence of Translocation>

### (Obtaining of Subject)

Blood was collected over time from a patient having developed acute myeloid leukemia (AML) due to a driver mutation of reciprocal translocation occurring between the KMT2A gene located on the long arm of chromosome 11 (11q23.3) and the AFDN gene located on the long arm of chromosome 6 (6p23) (translocation occurring between a nucleotide at position 118353449 on chromosome 11 and a nucleotide at position 168247755 on chromosome 6) (hereinafter referred to as the patient E) . The blood was collected with a blood collecting tube containing a coagulating agent four times in total, namely, immediately before starting treatment, 30 days after starting the treatment, 60 days after starting the treatment, and 68 days after starting the treatment. Subsequently, the blood held in the blood collecting tube was centrifuged, and thus a blood cell component was settled on the bottom of the blood collecting tube to collect a supernatant of a plasma . In Example 4, cfDNA and/or ctDNA present in the plasma was used as a template of digital PCR.

### [Detection of Structural Variation by Quantitative PCR]

### (Creation of Primer and Probe)

A first primer pair, a second primer pair, a first probe and a second probe to be used for detecting the translocation occurring between the KMT2A gene located on the long arm of chromosome 11 and the AFDN gene located on the long arm of chromosome 6, that is, the specific structural variation of the patient E, were created. The created primers and probes are shown in Table 5. It is noted that SEQ ID NO: 25 of the sequence listing corresponds to the forward primer of the first primer pair, SEQ ID NO: 26 corresponds to the reverse primer of the first primer pair, SEQ ID NO: 27 corresponds to the forward primer of the second primer pair, SEQ ID NO: 28 corresponds to the reverse primer of the second primer pair, SEQ ID NO: 29 corresponds to the first probe, and SEQ ID NO: 30 corresponds to the second probe.

**[Table 5]**

| | | Nucleotide sequence (5' to 3') |
|---|---|---|
| First primer pair | Forward (SEQ ID NO: 25) | TGCTTGAGGCCAGCAGTTC |
| | Reverse (SEQ ID NO: 26) | ACCATGCCTGGCTAACTTCTTC |
| Second primer pair | Forward (SEQ ID NO: 27) | TGCTTGAGGCCAGCAGTTC |
| | Reverse (SEQ ID NO: 28) | TTGAGGAGAGTGGTTTTCGTGA |
| First probe (SEQ ID NO: 29) | | TGGGCAACATAGCAAGACCCTGTCTTTATTTAA |
| Second probe (SEQ ID NO: 30) | | AGCCTGGGCAACATAGCAAGAGCTTTTAAC |

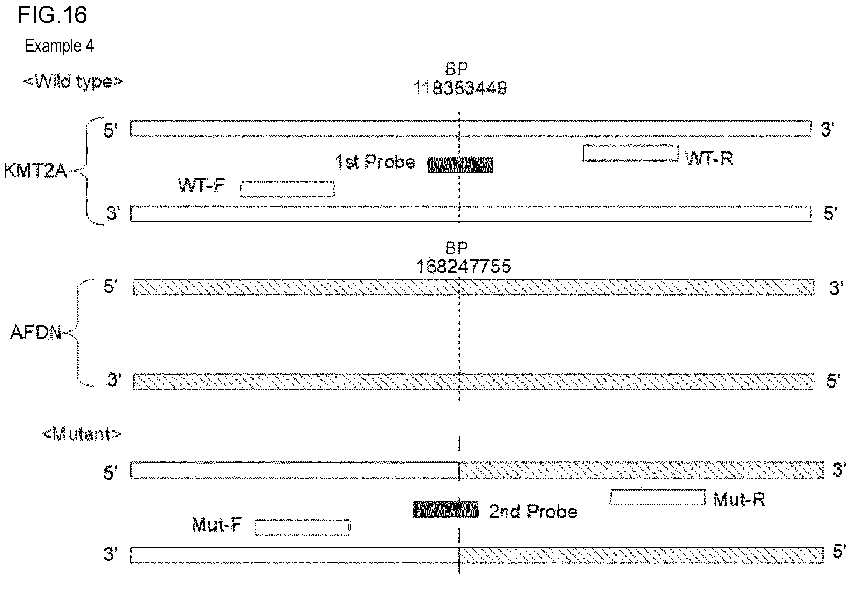

As illustrated in Fig. 16, the first primer pair was designed to sandwich a portion in KMT2A corresponding to the specific structural variation, namely, a breakpoint where the nucleotide sequence of KMT2A was broken on the occurrence of the translocation (BP in the drawing, the nucleotide at position 118353449) . On the other hand, the second primer pair was designed to allow the forward primer (Mut-F) to anneal to a sequence in "Mutant" corresponding to KMT2A, and the reverse primer (Mut-R) to anneal to a sequence corresponding to AFDN.

Besides, the first probe was designed to hybridize to a region, in the target sequence of the first primer pair, including the breakpoint where the nucleotide sequence of KMT2A was broken on the occurrence of the translocation (BP in the drawing, the nucleotide at position 118353449). On the other hand, the second probe was designed to hybridize to a region, in the target sequence of the second primer pair, including a joining point between sequences corresponding to KMT2A and AFDN.

Respective sequences of the primers and the probes were designed with Primer Express version 5.0 in the same manner as in Example 1, and were synthesized by FASMAC. Besides, also the probes were, in the same manner as in Example 1, modified with BHQ(R) 1 as a quencher at the 3' end, and the second probe was modified with FAM and the first probe was modified with HEX at the 5' end.

### (Digital PCR and Analysis of Results thereof)

Subsequently, the plasma collected from each of bloods collected immediately after starting the treatment, 30 days after starting the treatment, 60 days after starting the treatment, and 68 days after starting treatment was subjected to digital PCR using the primer pairs and the probes shown in Table 5. The digital PCR was performed in accordance with a method similar to that of Example 1. Before performing the digital PCR, nucleic acids such as cfDNA and ctDNA present in the plasma were concentrated by a method using magnetic beads, and a concentrate of the plasma obtained by the concentration was used in the digital PCR.

### (Analysis and Monitoring of Results of Digital PCR)

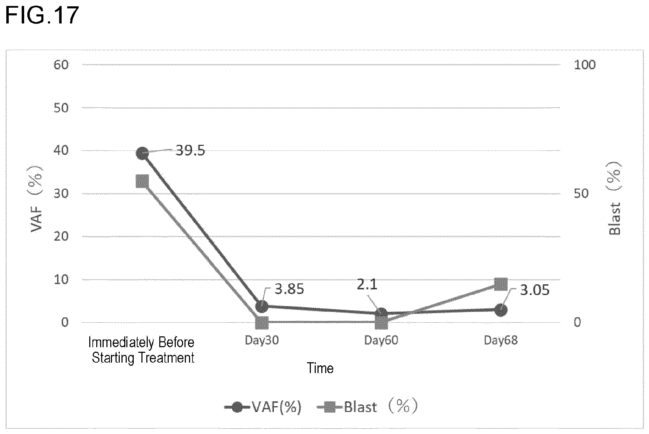

Subsequently, results of the digital PCR were analyzed. Fig. 17 is a graph having the abscissa indicating the chronological order in collecting the blood, and the ordinate indicating the variant allele frequency (VAF) . The VAF was calculated by a method similar to that of Example 3. It was revealed, based on the results illustrated in Fig. 17, that also when cfDNA and/or ctDNA contained in the blood collected from the patient E was used, the presence/absence of the mutant nucleotide sequence can be detected by employing the method according to one embodiment of the present invention. Besides, when the method according to one embodiment of the present invention is applied to bloods (subjects) collected over time, change over time in the presence/absence of the mutant nucleotide sequence, and change over time in the ratio between the mutant nucleotide sequence and the wild-type nucleotide sequence can be monitored.

Besides, Fig. 17 also illustrates a ratio (%) of blasts ("Blast") in leukocytes of each of the bloods collected over time as described above. A blast is considered as a cell that is highly possibly a leukemia cell, and for example, when the ratio of blasts in leukocytes is 20% or more in peripheral blood or bone marrow blood, the patient is diagnosed as acute leukemia. The change over time in the variant allele frequency obtained as the results of Example 4 shows substantially the same transition as the change over time of the ratio of blasts, and thus, the accuracy of the method according to one embodiment of the present invention was shown.

It is noted that the present invention is not limited to the embodiments and examples described above, but can be variously modified and changed within the scope of the appended claims. For example, in the above-described Examples, the analysis methods, the primer pair sets, and the method for designing primer pair sets suitable to the translocation occurring in the BCR gene and the ABL gene of the patients A and B, the deletion occurring in the FIP1L1-PDGFRA fusion gene of the patient C, the inversion occurring between the GATA2 gene and the MECOM gene of the patient D, and the translocation occurring in the KMT2A-AFDN fusion gene of the patient E are described, but the embodiments of the present invention are not limited thereto. The present invention can provide a primer pair coping with a structural variation of an individual subject, and an analysis method suitable for each structural variation by creating a probe corresponding to the primer pair.

## Claims

1. A method for analyzing the presence/absence of a specific structural variation in a sample DNA including a genomic DNA, a free DNA or a complementary DNA derived from a subject, comprising:
a detection step of detecting the presence/absence of the specific structural variationbyperforming PCR using, as a template, the sample DNA, and using, as a primer, a primer pair set consisting of a first primer pair and a second primer pair,
wherein the specific structural variation includes at least one selected from the group consisting of insertion, translocation, deletion, inversion, and tandem duplication, and the primer pair set is selected in accordance with the specific structural variation,
the first primer pair consists of a forward primer (WT-F) and a reverse primer (WT-R) that anneal at positions for amplifying a target sequence in a wild-type nucleotide sequence not including the specific structural variation,
the second primer pair consists of a forward primer (Mut-F) and a reverse primer (Mut-R) that anneal at positions for amplifying a target sequence in a mutant nucleotide sequence including the specific structural variation, and
the primer pair set includes one or more selected from the group consisting of:
a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
a primer pair set (D) consisting of the first primer pair designed so that the length of the target sequence to be amplified by the forward primer (WT-F) and the reverse primer (WT-R) when the specific structural variation is included is twice or more than that when the specific structural variation is not included, and the second primer pair designed so that the target sequence to be amplified by the forward primer (Mut-F) and the reverse primer (Mut-R) is present only when the specific structural variation is included.

2. The method according to claim 1, wherein when the specific structural variation is translocation occurring between two chromosomes, the forward primer (WT-F) and the reverse primer (WT-R) of the first primer pair in the primer pair set (B) anneal to only one of the two chromosomes.

3. The method according to claim 1, wherein the forward primer (WT-F) of the first primer pair and the forward primer (Mut-F) of the second primer pair are common primers having the same nucleotide sequence, or the reverse primer (WT-R) of the first primer pair and the reverse primer (Mut-R) of the second primer pair are common primers having the same nucleotide sequence.

4. The method according to claim 1, wherein the PCR performed in the detection step is quantitative PCR in which an amplification product is quantitatively determined using a nucleic acid probe that binds to a target sequence and has a labeling function, and the quantitative PCR is real-time PCR or digital PCR.

5. The method according to claim 4, wherein the nucleic acid probe includes one or more nucleic acid probe sets each consisting of a first probe that hybridizes to the target sequence of the first primer pair, and a second probe that hybridizes to the target sequence of the second primer pair,
the first probe and the second probe of the nucleic acid probe set are labeled by different fluorescent labels, and
in the quantitative PCR, an amount of amplification of the target sequence of the first primer pair and an amount of amplification of the target sequence of the second primer pair are both quantitatively determined by detecting fluorescence derived from the first probe and fluorescence derived from the second probe.

6. The method according to claim 5, wherein at least a part of the first probe is designed to hybridize to a region, in the target sequence of the first primer pair, having a sequence different from the target sequence of the second primer pair, and at least a part of the second probe is designed to hybridize to a region, in the target sequence of the second primer pair, having a sequence different from the target sequence of the first primer pair.

7. The method according to claim 6, comprising a calculation step of calculating a ratio between the wild-type nucleotide sequence and the mutant nucleotide sequence by comparing the amount of amplification of the target sequence of the first primer pair and the amount of amplification of the target sequence of the second primer pair based on results of the quantitative determination by the PCR.

8. The method according to claim 5, wherein when two or more primer pair sets and two or more nucleic acid probe sets are used in the quantitative PCR, the primer pair sets and the nucleic acid probe sets are in a relationship of one-to-one correspondence .

9. The method according to claim 1, comprising, when the sample DNA includes the genomic DNA or the free DNA, before the detection step, a whole genome sequence analysis step of performing whole genome sequence analysis on the sample DNA, and a determination step of determining the specific structural variation based on a result of the whole genome sequence analysis step.

10. The method according to claim 1, comprising, when the sample DNA includes the genomic DNA or the free DNA, a step of extracting the genomic DNA from the subject, or a step of isolating the free DNA from the subject.

11. The method according to claim 1, comprising a monitoring step of obtaining subjects from one individual over time, and monitoring change over time of a state of the specific structural variation of the individual by analyzing the present/absence of the specific structural variation in each sample DNA obtained from the subjects.

12. The method according to claim 1, wherein the subject includes blood, bone marrow, a body cavity fluid, a living body tissue, or a lymph tissue of a human or an animal.

13. The method according to claim 1, wherein the specific structural variation is a driver mutation of a disease.

14. The method according to any one of claims 1 to 13, wherein sequencing of an amplification product obtained by the PCR is not performed.

15. An MRD analysis method for analyzing minimal residual disease (MRD) by employing the method according to claim 1.

16. A primer pair set for use in PCR for analyzing the presence/absence of a specific structural variation in a sample DNA including a genomic DNA, a free DNA, or a complementary DNA derived from a subject,
the specific structural variation including at least one selected from the group consisting of insertion, translocation, deletion, inversion, and tandem duplication,
the primer pair set comprising a first primer pair and a second primer pair,
wherein the first primer pair consists of a forward primer (WT-F) and a reverse primer (WT-R) that anneal at positions for amplifying a target sequence in a wild-type nucleotide sequence not including the specific structural variation,
the second primer pair consists of a forward primer (Mut-F) and a reverse primer (Mut-R) that anneal at positions for amplifying a target sequence in a mutant nucleotide sequence including the specific structural variation, and
the primer pair set is one or more selected from the group consisting of:
a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
a primer pair set (D) consisting of the first primer pair designed so that the length of the target sequence to be amplified by the forward primer (WT-F) and the reverse primer (WT-R) when the specific structural variation is included is twice or more than that when the specific structural variation is not included, and the second primer pair designed so that the target sequence to be amplified by the forward primer (Mut-F) and the reverse primer (Mut-R) is present only when the specific structural variation is included.

17. A method for designing a primer pair set for use in PCR for analyzing the presence/absence of a specific structural variation in a sample DNA including a genomic DNA, a free DNA, or a complementary DNA derived from a subject,
wherein the specific structural variation includes at least one selected from the group consisting of insertion, translocation, deletion, inversion, and tandem duplication,
the primer pair set comprises a first primer pair and a second primer pair,
the first primer pair consists of a forward primer (WT-F) and a reverse primer (WT-R) that anneal at positions for amplifying a target sequence in a wild-type nucleotide sequence not including the specific structural variation,
the second primer pair consists of a forward primer (Mut-F) and a reverse primer (Mut-R) that anneal at positions for amplifying a target sequence in a mutant nucleotide sequence including the specific structural variation, and
the primer pair set is one or more selected from the group consisting of:
a primer pair set (A) consisting of the first primer pair designed so that a length of the target sequence to be amplified by the forward primer (WT-F) and the reverse primer (WT-R) when the specific structural variation is included is 10 times or more than that when the specific structural variation is not included, and the second primer pair designed so that the target sequence to be amplified by the forward primer (Mut-F) and the reverse primer (Mut-R) is present only when the specific structural variation is included;
a primer pair set (B) consisting of the first primer pair designed so that the target sequence to be amplified by the forward primer (WT-F) and the reverse primer (WT-R) is present only when the specific structural variation is not included, and the second primer pair designed so that the target sequence to be amplified by the forward primer (Mut-F) and the reverse primer (Mut-R) is present only when the specific structural variation is included;
a primer pair set (C) consisting of the first primer pair designed so that the target sequence to be amplified by the forward primer (WT-F) and the reverse primer (WT-R) is present only when the specific structural variation is not included, and the second primer pair designed so that the length of the target sequence to be amplified by the forward primer (Mut-F) and the reverse primer (Mut-R) when the specific structural variation is included is 1/10 or less than that when the specific structural variation is not included; and
a primer pair set (D) consisting of the first primer pair designed so that the length of the target sequence to be amplified by the forward primer (WT-F) and the reverse primer (WT-R) when the specific structural variation is included is twice or more than that when the specific structural variation is not included, and the second primer pair designed so that the target sequence to be amplified by the forward primer (Mut-F) and the reverse primer (Mut-R) is present only when the specific structural variation is included.
